(19) Europäisches Patentamt / European Patent Office / Office européen des brevets

(11) **EP 4 456 905 B1**

(12) **EUROPEAN PATENT SPECIFICATION**

(45) Date of publication and mention
of the grant of the patent:
**30.07.2025 Bulletin 2025/31**

(21) Application number: **22844182.0**

(22) Date of filing: **26.12.2022**

(51) International Patent Classification (IPC):
*A61K 35/741* (2015.01)   *A61K 35/747* (2015.01)
*A61P 1/16* (2006.01)

(52) Cooperative Patent Classification (CPC):
(C-Sets available)
**A61P 1/16; A61K 35/741; A61K 35/747**   (Cont.)

(86) International application number:
**PCT/EP2022/087841**

(87) International publication number:
**WO 2023/126384 (06.07.2023 Gazette 2023/27)**

(54) **PROBIOTIC BACTERIAL COMMUNITIES FOR USE IN THE TREATMENT OF A LIVER DISEASE**

PROBIOTISCHE BAKTERIENGEMEINSCHAFTEN ZUR VERWENDUNG BEI DER BEHANDLUNG EINER LEBERERKRANKUNG

COMMUNAUTÉS BACTÉRIENNES PROBIOTIQUES POUR UTILISATION DANS LE TRAITEMENT D'UNE MALADIE DU FOIE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **27.12.2021 EP 21217852**

(43) Date of publication of application:
**06.11.2024 Bulletin 2024/45**

(73) Proprietors:
• **MRM Health NV**
  **9052 Gent (BE)**
• **Universiteit Gent**
  **9000 Gent (BE)**

(72) Inventors:
• **VAN DE WIELE, Tom**
  **9000 Gent (BE)**
• **PINHEIRO, Iris**
  **9052 Gent (BE)**
• **POSSEMIERS, Sam**
  **9052 Gent (BE)**
• **BOLCA, Selin**
  **9052 Gent (BE)**
• **LOPES, Joana**
  **9052 Gent (BE)**
• **PANDA, Suchita**
  **9052 Gent (BE)**

(74) Representative: **Nederlandsch Octrooibureau**
**P.O. Box 29720**
**2502 LS The Hague (NL)**

(56) References cited:
**EP-A2- 3 690 026     WO-A1-2017/134240**
**US-A1- 2019 117 709**

• **TERESA GARCÍA-LEZANA ET AL: "Restoration of a healthy intestinal microbiota normalizes portal hypertension in a rat model of nonalcoholic steatohepatitis", HEPATOLOGY, JOHN WILEY & SONS, INC, US, vol. 67, no. 4, 19 February 2018 (2018-02-19), pages 1485 - 1498, XP071563871, ISSN: 0270-9139, DOI: 10.1002/ HEP.29646**

**(Cont. next page)**

- **YAO MINGFEI ET AL: "An Update on the Efficacy and Functionality of Probiotics for the Treatment of Non-Alcoholic Fatty Liver Disease", vol. 7, no. 5, 1 May 2021 (2021-05-01), pages 679 - 686, XP055929926, ISSN: 2095-8099, Retrieved from the Internet <URL:https://www.sciencedirect. com/science/article/pii/S2095809920302721/ pdfft? md5=5b3203468198137c2f3dae4e22e20c71& pid=1-s2.0-S2095809920302721-main.pdf> DOI: 10.1016/j.eng.2020.01.017**
- **EZZAIDI NIAMA ET AL: "New insights and therapeutic implication of gut microbiota in non-alcoholic fatty liver disease and its associated liver cancer", CANCER LETTERS, NEW YORK, NY, US, vol. 459, 8 June 2019 (2019-06-08), pages 186 - 191, XP085739657, ISSN: 0304-3835, [retrieved on 20190608], DOI: 10.1016/ J.CANLET.2019.114425**

Remarks:
    The complete document including Reference Table(s) and the Sequence Listing(s) can be downloaded from the EPO website

(52) Cooperative Patent Classification (CPC): (Cont.)

    C-Sets
    A61K 35/741, A61K 2300/00;
    A61K 35/747, A61K 2300/00

**Description**

**Field**

Aspects and embodiments described herein relate to the field of microbial compositions for human and animal health.

**Background**

Non-alcoholic fatty liver disease (NAFLD) is the most common chronic liver disease and its global prevalence is estimated to be around 25%, with the Middle East and South America reporting most cases, whereas Africa has the lowest prevalence.(1) NAFLD is considered the hepatic manifestation of the metabolic syndrome, and common comorbidities include obesity, type 2 diabetes, hyperlipidemia, and hypertension.(2) The prevalence of NAFLD and associated comorbidities is increasing (1,3), and as a result, NAFLD is rapidly becoming a major economical and social burden.(4) NAFLD can be further classified into non-alcoholic fatty liver (NAFL), also known as simple steatosis, and non-alcoholic steatohepatitis (NASH), characterized by the presence of hepatic inflammation. NASH is an aggressive form of the disease which can progress to hepatic fibrosis, cirrhosis, liver failure, and hepatocellular carcinoma (HCC).(2) Metabolic dysregulation is a hallmark of NAFLD. Insulin resistance, hyperlipidemia or dyslipidemia are common features, and therefore lipid modulators such as thiazolidinediones (TZDs) have been successfully tested in human trials for the treatment of NAFLD. However, significant side effects have been reported such as weight gain, fluid retention and risk of myocardial infarction.(6-8) In addition, so far, NASH has no approved pharmacotherapy.

Transfer of fecal matter collected from lean rats into NASH rats fed a high-fat glucose/fructose diet (HFGFD) has been described (9). Yet, no significant changes on histology were observed. The fact that fecal transfer or fecal microbial transplantation (FMT) is a poorly characterized procedure comes with transmission risks of infectious diseases and raises doubts about its applicability in less acute and life-threatening pathologies. Indeed, FMT is unlikely to become a long-term therapeutic option, especially considering the chronic and progressive nature of this disease.

WO2017/134240 describes a consortium consisting essentially of *Faecalibacterium prausnitzii, Butyricicocccus pullicaecorum, Roseburia inulinivorans, Roseburia hominis, Akkermansia mucinphila, Lactobacillus plantarum* and *Anaerostipes caccae,* and its use in gastro-intestinal disorders.

US 2019/117709 A1 relates to a method and system for reducing the likelihood of developing liver cancer in an individual diagnosed with non-alcoholic fatty liver disease and mentions a bacterial composition including *F. prausnitzii and Akkermansia muciniphila.*

EP 3690026 A2 describes effects of *Roseburia* strains on alcoholic fatty liver disease.

**Summary**

Any references in this description to methods of treating a disease, are to be interpreted as references to compounds, agents or compositions (as disclosed herein) for use in those methods.

In view of the above, there is still a need for improved means and methods which can be used for treating, delaying, ameliorating, preventing or curing liver pathologies or associated conditions and symptoms, and which do not have all the drawbacks of existing therapies.

It has been surprisingly found that specific combinations of bacteria have particular and surprising beneficial effects on the health of animals and humans. Particularly, as elaborated elsewhere herein and in the experimental part, it has been found that the compositions as described herein exert at least the following beneficial effects: reduction of weight gain, reduction of insulin levels, reduction of glucose levels, reduction of insulin resistance, reduction of portal pressure and intrahepatic vascular resistance, counteraction of endothelial dysfunction, reduction of liver inflammation, reduction of steatosis, reduction of hepatocellular ballooning and reduction of fibrosis, among others.

Accordingly, the aspects and embodiments described herein solve at least some of the problems and needs as discussed herein.

The invention is defined in the appended claims and relates to a composition comprising:

at least one bacterial strain of *Faecalibacterium prausnitzii;*
at least one bacterial strain of *Butyricicocccus pullicaecorum;*
at least one bacterial strain of *Roseburia inulinivorans;*
at least one bacterial strain of *Akkermansia muciniphila;*
at least one bacterial strain of *Lactiplantibacillus plantarum;* and
at least one bacterial strain of *Anaerostipes caccae,*
for use in a method of treating, delaying, ameliorating, preventing or curing non-alcoholic fatty liver disease (NAFLD), liver fibrosis, liver cirrhosis and/or hepatocellular carcinoma (HCC).

The invention also relates to a composition comprising bacterial strains belonging to the species *Faecalibacterium prausnitzii, Butyricicocccus pullicaecorum, Roseburia inulinivorans, Akkermansia muciniphila, Lactiplantibacillus plantarum, Anaerostipes caccae, Phocaeicola vulgatus, Veillonella parvula,* and *Blautia obeum.*

## DEPOSIT OF BIOLOGICAL MATERIAL

Purified cultures of the microbial strains LMG P-29362, LMG P29360, LMG P-29365, LMG P-29361, LMG P-29366 and LMG P29359 described in the present application were deposited by depositor Prodigest (Technologiepark 3, 9052 Zwijnaarde, Belgium; current address: Technologiepark 82, 9052 Zwijnaarde, Belgium) with BCCM/LMG Laboratorium voor Microbiologie, Universiteit Gent (UGent), K. L. Ledeganckstraat 35, B-9000 Gent, Belgium, recognized as an International Depositary Authority by the Budapest Treaty and the World Intellectual Property organization. Original deposits have been done on 18 January 2016.

## Description

The technical disclosure set out below may in some respects go beyond the scope of the claims. Elements of the disclosure which do not fall within the scope of the claims are provided for information.

Compositions

In an aspect useful for understanding the invention, there is provided a composition comprising two or more bacterial strains of at least two of the following genera: *Faecalibacterium, Butyricicocccus, Roseburia, Akkermansia, Lactiplantibacillus* and *Anaerostipes.* In other words, this means that the composition comprises minimally two bacterial strains, both from a different genus selected from *Faecalibacterium, Butyricicocccus, Roseburia, Akkermansia, Lactiplantibacillus* and *Anaerostipes.* Thus, for example, a composition comprising three bacterial strains may comprise two strains from one genus and a third strain from a different genus, or alternatively, three strains from a different genus. Preferably, said composition is for use in a method of treating, delaying, ameliorating, preventing or curing a disease or condition of the liver or a symptom thereof. Diseases and conditions of the liver and symptoms thereof are described in more detail elsewhere herein (see section "methods and uses").

The genus *Lactiplantibacillus* was previously included in the genus *Lactobacillus* (Zheng et al. Int J Syst Evol Microbiol 2020;70(4):2782-2858).

In some aspects useful for understanding the invention, a composition as described herein may comprise three or more, preferably four or more, or more preferably five or more bacterial strains of at least three, at least four resp. at least five of the following genera: *Faecalibacterium, Butyricicocccus, Roseburia, Akkermansia, Lactiplantibacillus* and *Anaerostipes.*

According to the invention, there is provided a composition comprising six or more bacterial strains of the following genera: *Faecalibacterium, Butyricicocccus, Roseburia, Akkermansia, Lactiplantibacillus* and *Anaerostipes.* Such compositions thus comprise at least one bacterial strain of each of said six genera. In some embodiments, a composition may comprise or consist essentially of one strain of each of said six genera.

In some aspects useful for understanding the invention, compositions provided herein are such that they comprise at least one bacterial strain belonging to the genus *Faecalibacterium* and at least one bacterial strain belonging to the genus *Anaerostipes.*

A bacterial strain, as used herein, may be understood to refer to a genetic variant or subtype of a microorganism. Several different strains can belong to the same bacterial species. Throughout this disclosure, the term "bacterial strain" can also be replaced with the term "bacterium" or "bacterial member". In any of the compositions provided herein, the bacterial strains may be isolated or purified, for example, from a source such as a culture or a microbiota sample (e.g., fecal matter). As used herein, the terms "isolated" and "purified" refers to a bacterium or bacterial strain or bacterial member or a composition thereof that has been separated from one or more associated substances found in a source material or any material associated with the bacteria in any process used to produce the preparation, such as another bacterium or bacterial strain, one or more components of a growth medium, and/or one or more components of a sample, such as a fecal sample. In some embodiments, the bacteria are "substantially isolated" or "substantially purified", for example from a source, such that other components of the source are not detected.

As described above, compositions described herein may comprise two, three, four, five, six or more bacterial strains belonging to the genera *Faecalibacterium, Butyricicocccus, Roseburia, Akkermansia, Lactiplantibacillus* and *Anaerostipes.* In some aspects useful for understanding the invention, the two, three, four, five, six or more bacterial strains are selected from particular species within said genera. According to the invention, a species of *Faecalibacterium* is *Faecalibacterium prausnitzii.* A species of *Butyricicocccus* is *Butyricicocccus pullicaecorum.* A species of *Roseburia* is *Roseburia inulinivorans. A* species of *Akkermansia* is *Akkermansia muciniphila.* A species of *Lactiplantibacillus* is *Lactiplantibacillus plantarum. A* species of *Anaerostipes* is *Anaerostipes caccae.*

The above-described species are well-known to a skilled person. In particular, the bacterial species *Faecalibacterium prausnitzii* (Duncan et al. Int J Syst Evol Microbiol. 2002;52: 2141-2146), *Butyricicoccus pullicaecorum* (Eeckhaut et al. Int J Syst Evol Microbiol 2008;58:2799-2802), *Roseburia inulinivorans* (Duncan et al. Int J Syst Evol Microbiol. 2006;56:2437-2441), *Akkermansia muciniphila* (Derrien et al. Int J Syst Evol Microbiol. 2004;54:1469-1476), *Lactiplantibacillus plantarum* (Walter. Appl Environ Microbiol 2008;74:4985-4996) and *Anaerostipes caccae* (Schwiertz et al. Syst Appl Microbiol 2002; 25:46-51) are well-known bacterial species to a skilled person. The species *Lactiplantibacillus plantarum* was previously known as *Lactobacillus plantarum* (Zheng et al. Int J Syst Evol Microbiol 2020;70(4):2782-2858).

Accordingly, in some aspects useful for understanding the invention, there is provided a composition comprising two or more bacterial strains of at least two of the following species: *Faecalibacterium prausnitzii, Butyricicocccus pullicaecorum, Roseburia inulinivorans, Akkermansia muciniphila, Lactiplantibacillus plantarum* and *Anaerostipes caccae.*

As indicated above, compositions described herein may, in some aspects useful for understanding the invention, comprise at least one bacterial strain belonging to the genus *Faecalibacterium* and at least one bacterial strain belonging to the genus *Anaerostipes.* Accordingly, in some aspects useful for understanding the invention, compositions described herein are such that they comprise at least one bacterial strain belonging to the species *Faecalibacterium prausnitzii* and at least one bacterial strain belonging to the genus *Anaerostipes caccae.*

In some aspects useful for understanding the invention, a composition as described herein may comprise three or more, preferably four or more, or more preferably five or more bacterial strains belonging to at least three, at least four resp. at least five of the following species: *Faecalibacterium prausnitzii, Butyricicocccus pullicaecorum, Roseburia inulinivorans, Akkermansia muciniphila, Lactiplantibacillus plantarum* and *Anaerostipes caccae.*

According to the invention, there is provided a composition comprising six or more bacterial strains belonging to the following species: *Faecalibacterium prausnitzii, Butyricicocccus pullicaecorum, Roseburia inulinivorans, Akkermansia muciniphila, Lactiplantibacillus plantarum* and *Anaerostipes caccae.*

Such compositions thus comprise at least one bacterial strain of each of said six species. In some embodiments, a composition may comprise or consist essentially of one strain of each of said six species.

As described above, compositions described herein may comprise two, three, four, five, six or more bacterial strains belonging to the species *Faecalibacterium prausnitzii, Butyricicocccus pullicaecorum, Roseburia inulinivorans, Akkermansia muciniphila, Lactiplantibacillus plantarum* and *Anaerostipes caccae.* In some aspects, the two, three, four, five, six or more bacterial strains are selected from particular strains within said species.

Preferred strains of *Faecalibacterium prausnitzii* are *Faecalibacterium prausnitzii* LMG P-29362 and *Faecalibacterium prausnitzii* DSMZ 17677, and strains having 16S rRNA or rDNA sequences showing at least 97% sequence identity to the 16S rRNA or rDNA sequence of at least one of said strains, for example SEQ ID NO: 4.

Preferred strains of *Butyricicoccus pullicaecorum* are *Butyricicoccus pullicaecorum* LMG P-29360 and *Butyricicoccus pullicaecorum* LMG24109, and strains having 16S rRNA or rDNA sequences showing at least 97% sequence identity to the 16S rRNA or rDNA sequence of at least one of said strains, for example SEQ ID NO: 6.

Preferred strains of *Roseburia inulinivorans* are *Roseburia inulinivorans* LMG P-29365 and *Roseburia inulinivorans* DSMZ 16841, and strains having 16S rRNA or rDNA sequences showing at least 97% sequence identity to the 16S rRNA or rDNA sequence of at least one of said strains, for example SEQ ID NO: 1.

Preferred strains of *Akkermansia muciniphila* are *Akkermansia muciniphila* LMG P-29361 and *Akkermansia muciniphila* DSMZ 22959, and strains having 16S rRNA or rDNA sequences showing at least 97% sequence identity to the 16S rRNA or rDNA sequence of at least one of said strains, for example SEQ ID NO: 2.

Preferred strains of *Lactiplantibacillus plantarum* are *Lactiplantibacillus plantarum* LMG P-29366 and *Lactiplantibacillus plantarum* ZJ316, and strains having 16S rRNA or rDNA sequences showing at least 97% sequence identity to the 16S rRNA or rDNA sequence of at least one of said strains, for example SEQ ID NO: 5.

Preferred strains of *Anaerostipes caccae* are *Anaerostipes caccae* LMG P-29359 and *Anaerostipes caccae* DSMZ 14662, and strains having 16S rRNA or rDNA sequences showing at least 97% sequence identity to the 16S rRNA or rDNA sequence of at least one of said strains, for example SEQ ID NO: 3.

In some embodiments, the above-mentioned strains having 16S rRNA or rDNA sequences showing at least 97% sequence identity to a particular 16S rRNA or rDNA sequence (such as SEQ ID NO: 1-6) may show 97.5%, 98%, 98.5%, 99% or 99.5% sequence identity to said 16S rRNA or rDNA (such as SEQ ID NO: 1-6).

As used herein, the term "16S ribosomal RNA" or "16S rRNA" refers to a nucleic acid sequence which is a component of the small prokaryotic ribosomal subunit (30S). The 16S rRNA is known to act as a scaffold defining the positions of the ribosomal proteins. The genes coding for it are referred to as 16S rRNA genes or 16S rDNA and are commonly used for phylogenetic studies, as they are known to be highly conserved sequences.

It is well known to a person skilled in the art that 16S rRNA or rDNA sequences can be deposited in online sequence databases, for example at GenBank (https://www.ncbi.nlm.nih.qgov/genbank/) and that they can be retrieved based on their unique accession number for use as reference 16S rRNA or rDNA sequence in evaluation of sequence homology, as for example described by Eeckhaut et al. Int J Syst Evol Microbiol 2008;58:2799-2802. GenBank accession numbers for the 16S rRNA sequences of some of the bacterial strains provided herein are listed below. These accession numbers can be used to retrieve the respective 16S rRNA sequences from http://www.ncbi.nlm.nih.qov/genbank/ for assessment of sequence homology.

*Roseburia inulinivorans* DSMZ 16841: AJ270473.3 (SEQ ID NO: 1)
*Akkermansia muciniphila* DSMZ 22959: AY271254.1 (SEQ ID NO: 2)
*Anaerostipes caccae* DSMZ 14662: AJ270487.2 (SEQ ID NO: 3)
*Faecalibacterium prausnitzii* DSMZ 17677: AJ270469.2 (SEQ ID NO: 4)
*Lactiplantibacillus plantarum* ZJ316: JN126052.1 (SEQ ID NO: 5)
*Butyricicoccus pullicaecorum* LMG 24109 HH793440.1 (SEQ ID NO: 6)

The above-indicated strains having accession numbers LMG P-29362, LMG P29360, LMG P-29365, LMG P-29361, LMG P-29366 and LMG P29359 have been deposited with BCCM/LMG Laboratorium voor Microbiologie, Universiteit Gent (UGent), K. L. Ledeganckstraat 35, B-9000 Gent, Belgium.
The above-indicated strains having accession numbers DSMZ 17677, LMG24109, DSMZ 16841, DSMZ 22959, ZJ316 and DSMZ 14662 have been deposited in public collections, have been described extensively and are easily accessible to skilled persons worldwide.

*Butyrate production*

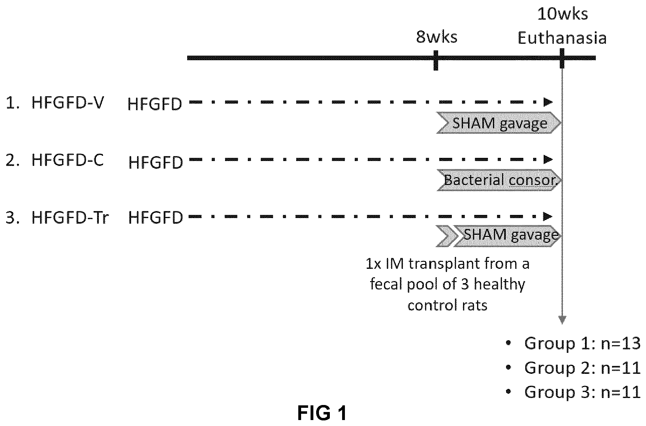

In some embodiments, a composition as described herein is such that it comprises at least one bacterial strain capable of producing butyrate. In some embodiments, a composition as described herein is such that it comprises at least two, at least three or at least four bacterial strains capable of producing butyrate.
In some embodiments, any of the two, three, four, five, six or more bacterial strains belonging to the genera *Faecalibacterium, Butyricicocccus, Roseburia, Akkermansia, Lactiplantibacillus* and *Anaerostipes,* as described elsewhere herein, may be a bacterial strain capable of producing butyrate. In some embodiments, at least one, two, three, four, five, six or more of the two, three, four, five, six or more bacterial strains of the genera *Faecalibacterium, Butyricicocccus, Roseburia, Akkermansia, Lactiplantibacillus* and *Anaerostipes,* as described elsewhere herein, may be a bacterial strain capable of producing butyrate. In some embodiments, all of the two, three, four, five, six or more bacterial strains belonging to the genera *Faecalibacterium, Butyricicocccus, Roseburia, Akkermansia, Lactiplantibacillus* and *Anaerostipes,* as described elsewhere herein, may be a bacterial strain capable of producing butyrate.
Bacterial strains belonging to the genera *Faecalibacterium, Butyricicocccus, Roseburia* and *Anaerostipes* are known to be capable of producing butyrate. In particular, *Faecalibacterium prausnitzii, Butyricicocccus pullicaecorum, Roseburia inulinivorans,* and *Anaerostipes caccae* are species known to be capable of producing butyrate.
*Roseburia inulinivorans* is known to produce both butyrate and propionate.
In some embodiments, provided herein are compositions as described above wherein further bacterial strains capable of butyrate production are added. In this context, commensal gut bacteria capable of butyrate production are preferred.
As used herein, a bacterial strain capable of butyrate production may be understood to refer to a bacterial strain carrying at least one of the pathways for butyrate production in its genome. Four major butyrate production pathways exist in bacteria (see for example Figure 1 in Vital et al. mBio 2014;5(2):e00889-14). These pathways utilize one of four substrates, namely, pyruvate, glutarate, 4-aminobutyrate and lysine. All pathways merge at a central energy-generating step where crotonyl-CoA is transformed to butyryl-CoA, catalyzed by the butyryl-CoA dehydrogenase electron-transfer-ring flavoprotein complex (Bcd-Etf$\alpha\beta$). The final production of butyrate is catalyzed by either butyryl-CoA:acetate CoA transferase (But) or butyrate kinase (Buk).
It is understood by the skilled person that, in any given experimental set-up, a bacterial strain capable of butyrate production as described herein may produce butyrate, or not, depending on the tested conditions including e.g. the growth medium.
Presence of these pathways in bacteria can be assessed by genetic methods known to the person skilled in the art, for example by the methods as described in Reichardt et al. The ISME Journal 2014;8:1323-1335. As an example, the presence of a marker gene can be detected by PCR or sequencing-based methods or using in silico phylogenetic tools. A suitable marker gene for the presence of the butyrate kinase pathway is butyrate kinase (Buk). A suitable marker gene for the presence of the butyryl-CoA:acetate CoA-transferase pathway is butyryl-CoA:acetate CoA-transferase (BCoAT or But). Alternatively, or additionally, butyrate production can be assessed by chemical analysis of *in vitro, in vivo* or *in situ* samples using methods known to a person of skill in the art, including gas chromatography (GC), liquid chromatography (LC), nuclear magnetic resonance (NMR), and capillary electrophoresis (CE).

*Propionate production*

As shown in the experimental part, it has been surprisingly found that the addition of at least one additional bacterial strain capable of producing propionate or a propionate precursor to the compositions described earlier herein is associated with specific advantages.

Accordingly, in some embodiments, a composition as described herein is such that it comprises at least one bacterial strain capable of producing propionate or a propionate precursor. In some embodiments, a composition as described herein is such that it comprises at least two, preferably at least three, more preferably at least four or more preferably at least five bacterial strains capable of producing propionate or a propionate precursor.

In some embodiments, any of the two, three, four, five, six or more bacterial strains belonging to the genera *Faecalibacterium, Butyricicocccus, Roseburia, Akkermansia, Lactiplantibacillus* and *Anaerostipes,* as described elsewhere herein, may be a bacterial strain capable of producing propionate or a propionate precursor. In some embodiments, at least one, two, three, four, five, six or more of the two, three, four, five, six or more bacterial strains of the genera *Faecalibacterium, Butyricicocccus, Roseburia, Akkermansia, Lactiplantibacillus* and *Anaerostipes,* as described elsewhere herein, may be a bacterial strain capable of producing propionate or a propionate precursor. In some embodiments, all of the two, three, four, five, six or more bacterial strains belonging to the genera *Faecalibacterium, Butyricicocccus, Roseburia, Akkermansia, Lactiplantibacillus* and *Anaerostipes,* as described elsewhere herein, may be a bacterial strain capable of producing propionate or a propionate precursor.

Bacterial strains belonging to the genera *Roseburia* and *Akkermansia* are known to be capable of producing propionate. In particular, *Roseburia inulinivorans* and *Akkermansia muciniphila* are species known to be capable of producing propionate.

*Roseburia inulinivorans* is known to produce both butyrate and propionate.

In some embodiments, a composition as described herein is such that it comprises at least one bacterial strain capable of producing butyrate and at least one bacterial strain capable of producing propionate or a propionate precursor. In some embodiments, a composition as described herein is such that it comprises at least two bacterial strains capable of producing butyrate and at least two bacterial strains capable of producing propionate or a propionate precursor. In some embodiments, a composition as described herein is such that it comprises at least three bacterial strains capable of producing butyrate and at least three bacterial strains capable of producing propionate or a propionate precursor. In some embodiments, a composition as described herein is such that it comprises at least four bacterial strains capable of producing butyrate and at least four bacterial strains capable of producing propionate or a propionate precursor. In some embodiments, a composition as described herein is such that it comprises at least four bacterial strains capable of producing butyrate and at least five bacterial strains capable of producing propionate or a propionate precursor.

Preferred compositions comprise at least four bacteria capable of producing butyrate and at least two bacteria capable of producing propionate or a propionate precursor. More preferred compositions comprise four bacteria capable of producing butyrate and three, preferably four, more preferably five bacteria capable of producing propionate or a propionate precursor.

It has been observed that in general the capacity to produce propionate or butyrate from sugars resides in different species, however, some exceptions are known. Notable exceptions that are capable of producing both butyrate and propionate are for example bacterial strains of *Roseburia inulinivorans.*

In some aspects useful for understanding the invention, any composition comprising two or more bacterial strains of at least two of the following genera: *Faecalibacterium, Butyricicocccus, Roseburia, Akkermansia, Lactiplantibacillus* or *Anaerostipes,* as described elsewhere herein, is such that the composition further comprises at least one additional bacterial strain capable of producing propionate or a propionate precursor. In some embodiments, a composition as described herein is such that the composition further comprises at least two additional bacterial strains capable of producing propionate or a propionate precursor. In some embodiments, a composition as described herein is such that the composition further comprises at least three additional bacterial strains capable of producing propionate or a propionate precursor. In preferred embodiments, a composition as described herein is such that the composition further comprises three additional bacterial strains capable of producing propionate or a propionate precursor.

In this context, commensal gut bacteria capable of propionate or propionate precursor production are preferred.

In some embodiments, the additional bacterial strain(s) capable of propionate or propionate precursor production belongs to the family *Veillonellaceae, Akkermansiaceae, Lachnospiraceae* or *Bacteroidetes,* preferably *Veillonellaceae, Lachnospiraceae* or *Bacteroidetes.*

A preferred genus in the *Veillonellaceae* family is *Veillonella.* Preferred species are *Veillonella parvula* and *Veillonella atypica.*

A preferred genus in the *Akkermansiaceae family* is *Akkermansia.* A preferred species is *Akkermansia*

*muciniphila.*

Preferred genera in the *Lachnospiraceae* family are *Blautia* and *Roseburia.* Preferred species within Blautia are *Blautia obeum, Blautia wexlerae,* and *Blautia luti.* A preferred species within *Roseburia* is *Roseburia inulinivorans.*

A preferred genus in the *Bacteroidetes* family is *Phocaeicola.*

A preferred species within the *Phocaeicola* is *Phocaeicola vulgatus.*

Accordingly, in some embodiments, the additional bacterial strain(s) capable of producing propionate or a propionate precursor as described herein is a bacterial strain of the genus *Veillonella, Akkermansia, Blautia, Roseburia* or *Phocaeicola.*

In preferred embodiments, the additional bacterial strain(s) capable of producing propionate or a propionate precursor as described herein is a bacterial strain of the genus *Phocaeicola, Veillonella* or *Blautia.*

In preferred embodiments, the additional bacterial strain(s) capable of producing propionate or a propionate precursor as described herein is a bacterial strain of species *Phocaeicola vulgatus, Veillonella parvula, Veillonella atypica, Blautia obeum, Blautia wexlerae,* or *Blautia luti.*

*Blautia wexlerae* and *Blautia luti* strains are known to produce the propionate precursor succinate.

In more preferred embodiments, a composition comprising two or more bacterial strains of at least two of the following genera: *Faecalibacterium, Butyricicocccus, Roseburia, Akkermansia, Lactiplantibacillus* or *Anaerostipes,* as described elsewhere herein, further comprises:

- *Phocaeicola vulgatus;*
- *Veillonella parvula* or *Veillonella atypica;* and
- *Blautia obeum, Blautia wexlerae,* or *Blautia luti.*

In some embodiments, a composition as described herein is a composition comprising bacterial strains belonging to the genera *Faecalibacterium, Butyricicocccus, Roseburia, Akkermansia, Lactiplantibacillus* and *Anaerostipes,* preferably belonging to the species *Faecalibacterium prausnitzii, Butyricicocccus pullicaecorum, Roseburia inulinivorans, Akkermansia muciniphila, Lactiplantibacillus plantarum* and *Anaerostipes caccae,* further comprising at least two additional bacterial strains capable of producing propionate or a propionate precursor. It is understood that such a composition may comprise a total of 8 strains (in case one strain of each genus/species is included) or may comprise a total of more than 8 strains (for example if more strains of one of the genera/species is included).

In some embodiments, the at least two additional bacterial strains capable of producing propionate or a propionate precursor may be three additional bacterial strains capable of producing propionate or a propionate precursor.

In some embodiments, the two or more additional bacterial strains capable of producing propionate or a propionate precursor may belong to the genus *Phocaeicola, Veillonella* or *Blautia,* preferably to the species *Phocaeicola vulgatus, Veillonella parvula, Veillonella atypica, Blautia obeum, Blautia wexlerae,* or *Blautia luti.*

In some embodiments, a composition as described herein is a composition comprising bacterial strains belonging to the genera *Faecalibacterium, Butyricicocccus, Roseburia, Akkermansia, Lactiplantibacillus* and *Anaerostipes,* preferably belonging to the species *Faecalibacterium prausnitzii, Butyricicocccus pullicaecorum, Roseburia inulinivorans, Akkermansia muciniphila, Lactiplantibacillus plantarum* and *Anaerostipes caccae,* wherein the composition further comprises:

- *Phocaeicola vulgatus;*
- *Veillonella parvula* or *Veillonella atypica;* and
- *Blautia obeum, Blautia wexlerae,* or *Blautia luti.*

In some embodiments, a composition as described herein is a composition comprising bacterial strains belonging to the genera *Faecalibacterium, Butyricicocccus, Roseburia, Akkermansia, Lactiplantibacillus, Anaerostipes* and *Phocaeicola,* and at least one of *Veillonella* or *Blautia.* Preferred is a composition comprising bacterial strains belonging to the species *Faecalibacterium prausnitzii, Butyricicocccus pullicaecorum, Roseburia inulinivorans, Akkermansia muciniphila, Lactiplantibacillus plantarum, Anaerostipes caccae* and *Phocaeicola vulgatus,* and at least one of *Veillonella parvula, Veillonella atypica, Blautia obeum, Blautia wexlerae,* or *Blautia luti.* In some instances, the bacterial members can include *Veillonella,* preferably *Veillonella parvula* or *Veillonella atypica.* In other instances, the bacterial members can include *Blautia,* preferably *Blautia obeum, Blautia wexlerae,* or *Blautia luti.* In yet other instances, the bacterial members can include both *Veillonella* and *Blautia,* preferably both *Veillonella parvula* or *Veillonella atypica;* and *Blautia obeum, Blautia wexlerae,* or *Blautia luti.*

In some embodiments, the additional bacterial strain(s) capable of producing propionate or a propionate precursor as described herein are selected from particular strains of the above-mentioned species. A preferred strain of *Phocaeicola vulgatus* is *Phocaeicola vulgatus* LMG17767, and strains having 16S rRNA or rDNA sequences showing at least 97% sequence identity to the 16S rRNA or rDNA sequence of said strain (SEQ ID NO: 7).

A preferred strain of *Veillonella parvula* is *Veillonella parvula* DSM 2007, and strains having 16S rRNA or rDNA sequences showing at least 97% sequence identity to the 16S rRNA or rDNA sequence of said strain (SEQ ID NO: 8).

A preferred strain of *Blautia obeum* is *Blautia obeum* DSM 25238, and strains having 16S rRNA or rDNA sequences showing at least 97% sequence identity to the 16S rRNA or rDNA sequence of said strain (SEQ ID NO: 9).

In some embodiments, the above-mentioned strains having 16S rRNA or rDNA sequences showing at least 97% sequence identity to a particular 16S rRNA or rDNA sequence (such as SEQ ID NO: 7-9) may show 97.5%, 98%, 98.5%, 99% or 99.5% sequence identity to said 16S rRNA or rDNA (such as SEQ ID NO: 7-9).

The above-indicated strains having accession numbers LMG17767, DSM 2007 and DSM 25238, have been deposited in public collections, have been described extensively and are easily accessible to skilled persons worldwide.

As used herein, a bacterial strain capable of propionate production may be understood to refer to a bacterial strain carrying at least one of the pathways for propionate production in its genome, such as the acrylate pathway, the propanediol pathway and the succinate pathway (see Figure 1 in Reichardt et al. The ISME Journal 2014;8:1323-1335).

As used herein, a bacterial strain capable of propionate precursor production may be understood to refer to a bacterial strain carrying at least one of the pathways for production of a propionate precursor in its genome. A propionate precursor may be any precursor or metabolic intermediate in any one of the different pathways of propionate production. In some embodiments, a propionate precursor as described herein may be selected from the group consisting of: acrylate, aspartate, fumarate, malate, 2-oxoglutarate, malate, oxaloacetic acid, and succinate. In preferred embodiments, a propionate precursor as described herein is succinate.

It is understood by the skilled person that, in any given experimental set-up, a bacterial strain capable of propionate or propionate precursor production as described herein may produce propionate or the propionate precursor, or not, depending on the tested conditions including e.g. the growth medium. This could be because, for example, some of the propionate production pathways require specific substrates. For example, some *Blautia* strains, such as, *Blautia obeum,* produce propionate from fucose.

Presence of propionate pathways in bacteria can be assessed by genetic methods known to the person skilled in the art, for example by the methods as described in Reichardt et al. The ISME Journal 2014;8:1323-1335. As an example, the presence of a marker gene can be detected by PCR or sequencing-based methods or using in silico phylogenetic tools. A suitable marker gene for the presence of the acrylate pathway is lactoyl-CoA dehydratase (IcdA). A suitable marker gene for the presence of the succinate pathway is methylmalonyl-CoA decarboxylase (mmdA). A suitable marker gene for the presence of the propanediol pathway is propionaldehyde dehydrogenase or propanol dehydrogenase (pduP, pduQ).

Alternatively, or additionally, propionate (or propionate precursor) production can be assessed by chemical analysis of *in vitro, in vivo* or *in situ* samples using methods known to a person of skill in the art, including gas chromatography (GC), liquid chromatography (LC), nuclear magnetic resonance (NMR), and capillary electrophoresis (CE).

Altogether, any bacterial strain capable of producing butyrate as described herein may be selected from the following genera: *Faecalibacterium, Butyricicocccus, Roseburia* and *Anaerostipes;* and the following species: *Faecalibacterium prausnitzii, Butyricicocccus pullicaecorum, Roseburia inulinivorans* and *Anaerostipes caccae*.

Similarly, any bacterial strain capable of producing propionate or a propionate precursor as described herein may be selected from the following families: *Veillonellaceae, Akkermansiaceae, Lachnospiraceae* and *Bacteroidetes;* the following genera: *Roseburia, Akkermansia, Veillonella, Blautia* and *Phocaeicola;* and the following species: *Roseburia inulinivorans, Akkermansia muciniphila, Phocaeicola vulgatus, Veillonella parvula, Veillonella atypica, Blautia obeum, Blautia wexlerae,* and *Blautia luti.*

*Further components*

In a further aspect, the compositions described herein may further comprise one or more prebiotics. The term 'prebiotic' refers to any chemical that is capable of inducing the growth or activity of microorganisms (e.g., bacteria) that contribute to the well-being of their host. Hence, prebiotics can influence or alter the composition of organisms in the gut microbiome. However, in principle it is a more general term that can refer to other areas of the body as well. Typically - but non-limiting - prebiotics are non-digestible fiber compounds that at least partially pass undigested through the upper part of the gastrointestinal tract and stimulate the growth or activity of advantageous bacteria that colonize the large bowel by acting as substrate for them.

In a further aspect, the compositions described herein may further comprise one or more therapeutic agents. In some embodiments, said therapeutic agent is selected from statins (e.g. simvastatin), other lipid lowering therapies, non-lipid lowering therapies, GLP-1 receptor agonists (e.g. semaglutide, dulaglutide, liraglutide), SGLT1/2 inhibitors (e.g. licoglifozin), SGLT2 inhibitors, DPP-4 inhibitors (e.g. itagliptin, saxagliptin, linagliptin, alogliptin), FXR agonists (e.g. OCA, tropifexor, cilofexor), inhibitors of apoptosis (e.g. selonsertib, emricasan), anti-inflammatory agents (e.g. pentoxifylline), anti-hypertensive drugs such as angiotensin receptor blockers, CCR2/5 antagonists (e.g. cenicriviroc), ACC inhibitors (e.g. firsocostat), anti-oxidative stress agents (e.g. vitamin E, UDCA) and PPAR agonists such as thiazolidinediones (TZDs) (e.g. pioglitazone).

In preferred embodiments, said therapeutic agent is selected from statins (e.g. simvastatin), GLP-1 receptor agonists (e.g. semaglutide, dulaglutide, liraglutide), SGLT2 inhibitors and PPAR agonists such as thiazolidinediones (e.g. pioglitazone). A preferred example of a GLP-1 receptor agonist is liraglutide. In preferred embodiments, said therapeutic agent is a PPAR agonist such as a thiazolidinedione (also known as glitazones) (e.g. pioglitazone).

*Specific compositions and derived products*

In some embodiments, the compositions described herein may be pharmaceutical compositions, i.e. a composition further comprising pharmaceutically acceptable ingredients.

As used herein, "pharmaceutically acceptable ingredients" include pharmaceutically acceptable carriers, fillers, preservatives, solubilizers, vehicles, diluents and/or excipients. Accordingly, the one or more pharmaceutically acceptable ingredients may be selected from the group consisting of pharmaceutically acceptable carriers, fillers, preservatives, solubilizers, vehicles, diluents (e.g. starch, cellulose derivatives or sugar derivatives) and/or excipients. Such pharmaceutically acceptable carriers, fillers, preservatives, solubilizers, vehicles, diluents and/or excipients may for instance be found in Remington: The Science and Practice of Pharmacy, 23rd edition. Elsevier (2020).

The compositions described herein may be formulated to provide a rapid and efficient therapeutic effect. In some embodiments, a composition as described herein may be formulated for delivery to the intestines, for example formulated as a composition for rectal administration or for oral administration, preferably for oral administration.

In some embodiments, the compositions described herein may be formulated as a capsule, microcapsule, tablet, granule, powder, troche, pill, food supplement, suspension, suppository or syrup and the like, preferably a powder, a suspension, a tablet or a food supplement. In some embodiments, the compositions as described herein may be provided in a suspension dosage form.

In some embodiments, the (pharmaceutical) compositions described herein may be formulated as a composition for rectal administration. In this regard, any of the compositions described herein can be provided as a (pharmaceutical) dosage form for rectal administration, such as a suppository.

In some embodiments, the (pharmaceutical) compositions described herein may be formulated as a composition for oral administration, such as a capsule, microcapsule, tablet, granule, powder, troche, pill, food supplement, suspension or syrup.

In some embodiments, the compositions described herein may comprise lyophilized bacteria or bacteria provided in an active culture format. In preferred embodiments, the bacteria may be lyophilized.

In some embodiments, the compositions described herein can be incorporated in a food product, beverage, dietary supplement or nutraceutical. Aspects of the present disclosure thus also provide food products, beverages, dietary supplements and nutraceuticals comprising any of the compositions described herein. Also within the scope of the present disclosure are food products, beverages, dietary supplements and nutraceuticals comprising any (combinations) of the bacterial strains described herein. A "food product" is typically an edible material composed primarily of one or more of the macronutrients protein, carbohydrate and fat. A food may also contain one or more micronutrients such as vitamins or minerals. Examples of foods in which the composition may be incorporated include snack bars, cereals, buns, muffins, biscuits, cakes, pastries, processed vegetables, sweets, probiotic formulations including yoghurts, beverages, plant oil-based liquids, animal fat-based liquids, frozen confections and cheeses. Preferred foods include yoghurts, cheeses and other dairy products.

Examples of beverages include soft beverages, syrups, squashes, dry drink mixes and nutritional beverages.

A nutraceutical is a food ingredient, food supplement or food product which is considered to provide a medical or health benefit, including the prevention and treatment of disease. Nutraceuticals are sometimes also denoted as "functional food", i.e. a food that is typically marketed as providing a health benefit beyond that of supplying pure nutrition to the consumer.

The disclosure also provides a probiotic comprising a composition discussed herein. A probiotic is typically a live supplement which can enhance the intestinal microbiota. Such probiotics may be given in particularly to humans but also to farm and domestic animals and to aquatic organisms. The probiotic may additionally comprise one or more acceptable excipients or flavorings, which are suitable for ingestion by a human or animal.

In some embodiments, the compositions as described herein comprise at least $10^2$, preferably at least $10^3$, more preferably at least $10^4$, even more preferably at least $10^5$ CFU of bacteria. In some embodiments, the compositions as described herein comprise between $10^2$ and $10^{14}$, preferably between $10^3$ and $10^{13}$, more preferably between $10^4$ and $10^{12}$, even more preferably between $10^5$ and $10^{11}$ colony forming units (CFU) of bacteria. In some embodiments, the compositions as described herein comprise at least $10^5$ colony forming units of bacteria, for example between $10^5$ and $10^{11}$ colony forming units of bacteria.

It is understood that, with respect to any of the compositions provided throughout this disclosure, the term "comprising" may be replaced with the term "consisting essentially of" or "consisting". In other words, in some embodiments, the compositions provided herein consist essentially of the bacterial strains as provided, or consist of the bacterial strains as provided.

In addition to the compositions as described herein, a preferred aspect of this disclosure also relates to the same compositions for use in any of the methods described later herein (see section "methods and uses").

Methods for obtaining compositions; and compositions obtained thereby

In some embodiments, the compositions as provided herein may be obtained by the methods described in this section. In some embodiments, the compositions as provided herein may be obtainable by the methods described in this section. In other words: in some embodiments, the bacterial strains comprised in the compositions provided herein are previously subjected to the methods described in this section. In this context, it can also be said that the bacterial strains are "preadapted" or "conditioned" by any of the methods described in this section.

The methods provided in this section impart certain advantageous characteristics on the compositions of this disclosure. In particular, the onset of the effect of the composition on a host will be quicker, and/or the effectiveness may be higher, i.e. they provide for a rapid and efficient therapeutic effect.

More specifically, the "preadapted" or "conditioned" compositions may significantly decrease the time of biotherapeutic onset meaning that, by being pre-adapted, the set of microorganisms can exert their functionality at least 5% quicker (on a temporal scale), preferably at least 10% quicker, more preferably at least 20% quicker and most preferably at least 30 % quicker as compared to a loosely assembled set of the same strains. The "preadapted" or "conditioned" compositions may also significantly increase the effect of treatment meaning that, by being preadapted, the set of microorganisms can exert their functionality with at least a 5% higher effectiveness, preferably at least 10% higher effectiveness, more preferably at least 20% and most preferably at least 30 % higher effectiveness. The effectiveness depends on the endpoint for which the set of microorganisms has been designed. Possible functionalities include but are not limited to reducing weight gain, improving insulin levels, reducing glucose levels, reducing insulin resistance, reducing portal pressure and intrahepatic vascular resistance, counteracting endothelial dysfunction, reducing liver inflammation, reducing steatosis, reducing hepatocellular ballooning and reducing fibrosis, among others.

In an aspect, there is provided a method for obtaining a composition as described herein, comprising growing the bacterial strains together in a reactor.

In some embodiments, said reactor is under standardized conditions representative for the gastro-intestinal tract.

The parameters characterizing the standardized conditions include but are not limited to: pH (range between 1.5 and 8); availability of carbon sources (either carbohydrate or proteins or a combination thereof); retention time in a specific reactor (range between 10 min and 200 h); oxygen availability (range between 0 and 8 g/L); availability of micronutrients; presence/absence of antibiotics; concentration of bile salts (range between 0 and 20 mM); presence of heavy metals; presence of host factors as immune molecules. In a preferred embodiment, the parameters characterizing the standardized conditions comprise pH, retention time in a specific reactor and concentration of bile salts, all as earlier defined herein. Depending on the complexity of the consortium, a period of 1 to 15 days may be suitable to obtain a functionally stable consortium. On average, in order to develop a consortium composed of 7 to 14 members, a time between 3 and 10 days is suitable to obtain a functionally stable consortium (depending on the environmental conditions). A composition as defined herein is therefore obtainable after having been trained or cultured during a time between 3 and 10 days under conditions wherein pH, retention time in a specific reactor and concentration of bile salts have been set as defined herein. Such a process allows the production of a composition or consortium which is functionally stable.

As used herein, "a functionally stable consortium" may be a composition as described herein still comprising the initial different number of species of bacteria after at least 3 or 5 or 10 days of culture.

In a further aspect, there is provided a reactor operating under standardized conditions representative for the GI tract, comprising: pH range between 1.5 and 8; availability of carbon sources; retention time between 10 min and 200 h; oxygen availability between 0 and 8 g/L; availability of micronutrients; presence/absence of antibiotics; concentration of bile salts between 0 and 20 mM; presence of heavy metals; presence of host factors as immune molecules. In an embodiment, said reactor is such that the parameters characterizing the standardized conditions comprise pH, retention time in a specific reactor and concentration of bile salts as defined in the previous paragraph. In an embodiment, such reactor comprises a composition of between 5 and 20 distinct bacteria members, or 6 to 14 distinct bacteria members or 5 to 15 distinct bacteria members. In a preferred embodiment, such composition resides for a time between 3 and 14 days or 3 and 10 days in such a reactor to obtain a functionally stable consortium.

Hence, within the context of compositions for use, methods and uses as described herein, the treatment may result in a faster biotherapeutic onset and/or increased effectiveness, i.e. rapid and efficient therapeutic effect, as described herein.

Methods and uses

As elaborated in the experimental part, it has been surprisingly found that the above-described compositions exert several direct and indirect beneficial effects on the liver. Specifically, compositions as described herein exert at least the following beneficial effects: reduction of weight gain, improvement of insulin levels, reduction of glucose levels, reduction of insulin resistance, reduction of portal pressure and intrahepatic vascular resistance, counteraction of endothelial dysfunction, reduction of liver inflammation, reduction of steatosis, reduction of hepatocellular ballooning and reduction of fibrosis, among others. It therefore follows that the compositions provided herein have broad applicability in treating, delaying, ameliorating, preventing or curing diverse diseases and conditions of the liver, as well as symptoms thereof, underlying diseases or conditions and extrahepatic manifestations.

Accordingly, in a further aspect useful for understanding the invention , the compositions as described herein are provided for use in medicine. Preferably, the compositions as described herein are for use in a method of treating, delaying, ameliorating, preventing or curing a disease or condition of the liver. It is also encompassed herein to treat symptoms of a disease or condition of the liver, diseases or conditions underlying the disease or condition of the liver, as well as extrahepatic manifestations of the disease or condition of the liver, each of which aspects are described in more detail elsewhere herein. Accordingly, in some aspects useful for understanding the invention, the compositions as described herein are provided for use in a method of treating, delaying, ameliorating, preventing or curing a disease or condition of the liver or a symptom thereof. In some aspects useful for understanding the invention, the compositions as described herein are provided for use in a method of treating, delaying, ameliorating, preventing or curing a disease or condition of the liver or a symptom thereof, an underlying disease or condition, or an extrahepatic manifestation.

In a further aspect useful for understanding the invention there is provided a method for treating, delaying, ameliorating, preventing or curing a disease or condition of the liver comprising administering a composition as described herein. In some aspects useful for understanding the invention there is provided a method for treating delaying, ameliorating, preventing or curing a disease or condition of the liver or a symptom thereof comprising administering a composition as described herein. In some aspects useful for understanding the invention there is provided a method for treating delaying, ameliorating, preventing or curing a disease or condition of the liver, a symptom thereof, an underlying disease or condition, or an extrahepatic manifestation comprising administering a composition as described herein. In some aspects useful for understanding the invention, administering a composition means administering said composition to a subject, such as a subject in need thereof. In a preferred embodiment, an effective amount or a therapeutically effective amount of a composition is administered.

In a further aspect useful for understanding the invention there is provided a use of a composition as described herein, for the manufacture of a medicament for treating, delaying, ameliorating, preventing or curing a disease or condition of the liver. In some aspects useful for understanding the invention there is provided a use of a composition as described herein, for the manufacture of a medicament for treating, delaying, ameliorating, preventing or curing a disease or condition of the liver or a symptom thereof. In some aspects useful for understanding the invention there is provided a use of a composition as described herein, for the manufacture of a medicament for treating, delaying, ameliorating, preventing or curing a disease or condition of the liver, a symptom thereof, an underlying disease or condition or an extrahepatic manifestation.

In a further aspect useful for understanding the invention, there is provided a use of a composition as described herein, for treating, delaying, ameliorating, preventing or curing a disease or condition of the liver. In some aspects useful for understanding the invention there is provided a use of a composition as described herein, for treating, delaying, ameliorating, preventing or curing a disease or condition of the liver or a symptom thereof. In some aspects useful for understanding the invention there is provided a use of a composition as described herein, for treating, delaying, ameliorating, preventing or curing a disease or condition of the liver, a symptom thereof, an underlying disease or condition or an extrahepatic manifestation.

Within the context of compositions for use, methods and uses provided herein, an effective amount or therapeutically effective amount of the composition as described herein may be administered.

As used herein, an "effective amount" is an amount sufficient to exert beneficial or desired results. Accordingly, a "therapeutically effective amount" is an amount that, when administered to a subject in need thereof, is sufficient to exert some therapeutic or prophylactic effect as described herein, such as, but not limited to, reduction of weight gain, improvement of insulin levels, reduction of glucose levels, reduction of insulin resistance, reduction of portal pressure and intrahepatic vascular resistance, counteraction of endothelial dysfunction, reduction of liver inflammation, reduction of steatosis, reduction of hepatocellular ballooning and reduction of fibrosis compared to an untreated subject. An amount that is "therapeutically effective" may vary from subject to subject, for example depending on the age, the disease progression or the overall general condition of the individual. An appropriate "therapeutically effective" amount in any individual case may be determined by the skilled person using routine experimentation, such as the methods described later herein, and/or the methods of the experimental part herein. It is understood that "effective amount" and "therapeutically effective amount" refer to the combined total amount of bacterial strains in the composition. Depending on the final application, the combined total amount can be the result of equal amounts of each of the bacterial strains or unequal amounts of each of the bacterial strains, in which each single strain has a minimum abundance of 0.0001% of the combined total amount, preferably a minimum abundance of 0.001% of the combined total amount and more preferably a minimum abundance of 0.01% of the combined total amount.

Depending on the final application, said effective amount or therapeutically effective amount (which is understood to refer to the combined total amount of bacteria) can be at least $10^2$, preferably at least $10^3$, more preferably at least $10^4$, even more preferably at least $10^5$ colony forming units (CFU) of bacteria. In some embodiments, said effective amount or therapeutically effective amount (which is understood to refer to the combined total amount of bacteria) can be between $10^2$ and $10^{14}$, preferably between $10^3$ and $10^{13}$, more preferably between $10^4$ and $10^{12}$, even more preferably between $10^5$ and $10^{11}$ colony forming units (CFU) of bacteria. In some embodiments, said amount is a daily amount, i.e. an amount that is administered daily.

A composition as described herein can be administered according to any regime. In some embodiments, the composition can be administered as a single one-time dose. In preferred embodiments, the composition can be administered to the subject as routinely or periodically, for example, the composition can be administered to the subject once every 12 hours, 24 hours, or 48 hours; i.e. two times per day, daily or once every two days. In preferred embodiments, the composition may be administered daily. In some embodiments, a composition as described herein is administered for a period of at least 1 week, 8 days, 9 days, 10 days, 11 days, 12 days, 13 days, 2 weeks, 3 weeks, 4 weeks, 5 weeks, 6 weeks, 7 weeks, 8 weeks, 9 weeks, 10 weeks, 11 weeks, 12 weeks, 3 months, 4 months, 5 months, 6 months, a year or more. In some embodiments, a composition as described herein is administered for life.

A "subject" or "subject in need" as used herein may be a human (preferred) or a non-human animal. In some embodiments, the subject (in need) can be a healthy subject, especially in the context of preventing a disease or condition of the liver or a symptom thereof as described herein. In some embodiments, the subject (in need) may also suffer from or be at risk for developing any of the diseases and conditions described herein. In some embodiments, the subject (in need) may be a subject inflicted with NASH.

Within the context of compositions for use, methods and uses provided herein, the composition may be administered to a subject in need as described herein.

Within the context of compositions for use, methods and uses provided herein, a composition may be administered by different administration modes. In some embodiments, an administration mode may be rectally or orally. Oral administration is preferred.

A composition as provided herein may be directly or indirectly administered using suitable means known in the art. Improvements in means for providing an individual with a composition as described herein are anticipated, considering the progress that has already thus far been achieved. Such future improvements may of course be incorporated to achieve the desired effects as described herein.

Within the context of compositions for use, methods and uses provided herein, a composition as described herein may be used in combination with one or more therapeutic agents. In some embodiments, said therapeutic agent is selected from statins (e.g. simvastatin), other lipid lowering therapies, non-lipid lowering therapies, GLP-1 receptor agonists (e.g. semaglutide, dulaglutide, liraglutide), SGLT1/2 inhibitors (e.g. licoglifozin), SGLT2 inhibitors, DPP-4 inhibitors (e.g. itagliptin, saxagliptin, linagliptin, alogliptin), FXR agonists (e.g. OCA, tropifexor, cilofexor), inhibitors of apoptosis (e.g. selonsertib, emricasan), anti-inflammatory agents (e.g. pentoxifylline), anti-hypertensive drugs such as angiotensin receptor blockers, CCR2/5 antagonists (e.g. cenicriviroc), ACC inhibitors (e.g. firsocostat), anti-oxidative stress agents (e.g. vitamin E, UDCA) and PPAR agonists such as thiazolidinediones (TZDs) (e.g. pioglitazone).

In preferred embodiments, said therapeutic agent is selected from statins (e.g. simvastatin), GLP-1 receptor agonists (e.g. semaglutide, dulaglutide, liraglutide), SGLT2 inhibitors and PPAR agonists such as thiazolidinediones (e.g. pioglitazone). A preferred example of a GLP-1 receptor agonist is liraglutide. In preferred embodiments, said therapeutic agent is a PPAR agonist such as a thiazolidinedione (also known as glitazones) (e.g. pioglitazone).

The compositions provided herein and the one or more therapeutic agents may be administered in a single composition or separately.

*Diseases and conditions of the liver*

In some aspects useful for understanding the invention, within the context of compositions for use, methods and uses provided herein, the disease or condition of the liver may be a liver disease or condition associated with disbyosis.

In some aspects useful for understanding the invention, within the context of compositions for use, methods and uses provided herein, the disease or condition of the liver may be alcohol-related liver disease (ARLD), cholestatic liver disease, non-alcoholic fatty liver disease (NAFLD), liver fibrosis, liver cirrhosis and/or a liver neoplasm or a symptom thereof. Alcohol-related liver disease (ARLD) encompasses alcoholic fatty liver disease, alcoholic hepatitis and (alcohol-related) cirrhosis. Non-limiting examples of cholestatic liver disease are primary biliary cholangitis (PBC) and primary sclerosing cholangitis (PSC).

According to the invention, within the context of compositions for use, methods and uses provided herein, the disease or condition of the liver may be non-alcoholic fatty liver disease (NAFLD), liver fibrosis, liver cirrhosis and/or a liver neoplasm or a symptom thereof.

NAFLD is now recognized as the hepatic manifestation of the metabolic syndrome. Accordingly, "NAFLD" as used in this disclosure may be replaced with terms such as the "liver component of metabolic syndrome", the "hepatic manifestation of metabolic syndrome", or "Metabolic (Dysfunction) Associated Fatty Liver Disease" (MAFLD) (as proposed in Eslam M et al. A new definition for metabolic dysfunction-associated fatty liver disease: an international expert consensus statement. J Hepatol. 2020; 73:202-209).

NAFLD is considered an umbrella term for a spectrum of liver diseases that can be classified as nonalcoholic fatty liver (NAFL) and nonalcoholic steatohepatitis (NASH) (see Lackner C. (2018) Nonalcoholic Fatty Liver Disease. Practical Hepatic Pathology: a Diagnostic Approach, 167-187). Thus, in some embodiments within the context of compositions for use, methods and uses provided herein, the disease or condition of the liver may be non-alcoholic fatty liver (NAFL), non-alcoholic steatohepatitis (NASH), liver fibrosis, liver cirrhosis and/or a liver neoplasm, a symptom thereof, or an underlying disease or condition.

The term "liver neoplasm" as used herein may be understood to refer to both liver neoplasms and intrahepatic bile duct neoplasms. Accordingly, in some embodiments, the term "liver neoplasm" or the like may be replaced with the term "liver or intrahepatic bile duct neoplasm" or the like.

According to the invention, a liver neoplasm as described herein is a liver cancer. According to the invention, a liver cancer is hepatocellular carcinoma (HCC).

Within the context of compositions for use, methods and uses provided herein, a method for treating, delaying, ameliorating, preventing or curing a disease or condition of the liver may be a method of delaying or preventing disease progression of NAFL, preferably delaying progression of NAFL to NASH, NASH to liver fibrosis, liver fibrosis to liver cirrhosis and/or liver cirrhosis to HCC, more preferably delaying NAFL to NASH or NASH to liver fibrosis.

Within the context of compositions for use, methods and uses provided herein, "treating, delaying, ameliorating, preventing or curing" as used herein may be understood to treat, delay, ameliorate, prevent or cure a disease, condition or symptom as compared to said disease, condition or symptom prior to administering the composition. In some embodiments, "treating, delaying, ameliorating, preventing or curing" as used herein may be understood to treat, delay, ameliorate, prevent or cure a disease, condition or symptom as compared to said disease, condition or symptom in a subject inflicted with the same disease, condition or symptom receiving a placebo. Similarly, the occurrence of any of the effects described elsewhere herein may be compared in the same way.

*Symptoms*

In some embodiments, a symptom of a disease or condition of the liver as described herein comprises weight gain, insulin resistance, liver inflammation, steatosis, hepatocellular ballooning, intrahepatic vascular resistance (IHVR), portal hypertension, endothelial dysfunction and/or fibrosis.

Within the context of compositions for use, methods and uses provided herein, a composition and/or a medicament as described herein preferably exhibits at least one, at least two, at least three, or all of the following effects:

- reducing weight gain;
- improving insulin levels;
- reducing glucose levels;
- reducing insulin resistance;
- reducing portal pressure;
- reducing intrahepatic vascular resistance;
- counteracting endothelial dysfunction;
- reducing liver inflammation
- reducing steatosis;
- reducing ballooning; and
- reducing fibrosis

Means and methods for measuring these symptoms and effects are well-known in the art. Non-limiting examples of methods for measuring these symptoms and effects are provided in the experimental section.

In this context, "reducing" (respectively "improving") and the like means at least a detectable decrease (respectively a detectable improvement) using an assay known to a person of skill in the art, such as assays as carried out in the experimental part. The reduction may be a decrease of at least 5%, at least 10%, at least 20%, at least 30%, at least 40%, at least 50%, at least 60%, at least 70%, at least 80%, at least 90% or at least 100%. In some embodiments, administering a composition as described herein can result in reduced body weight gain, improved portal hypertension, or improved insulin sensitivity, optionally as compared to a subject with a comparable liver disease or condition that is administered a placebo. In some embodiments, administering a composition as described herein can result in a reduction of about 40% in fasting blood insulin levels within two weeks of treatment, optionally as compared to a subject that is administered a placebo. In some embodiments, administering a composition as described herein can result in a reduction of about 80% in intrahepatic vascular resistance (IHVR) within 10 weeks of treatment, optionally as compared to a subject administered a placebo.

A non-alcoholic fatty liver disease (NAFLD) activity score (NAS) has been defined by the Pathology Committee of the NASH Clinical Research Network as the unweighted sum of scoring values for steatosis (ranging from 0-3), lobular inflammation (ranging from 0-3) and ballooning (ranging from 0-2), hence providing a NAS score ranging from 0 to 8. (See Kleinen et al., Design and Validation of a Histological Scoring System for Nonalcoholic Fatty Liver Disease, Hepatology, Vol. 41, No. 6, 2005, pp. 1313-1321. See in particular Table 1 therein). A fibrosis score ranging from 0-4 is also defined by Kleinen et al., see in particular Table 1 therein.

Within the context of compositions for use, methods and uses provided herein, a composition and/or a medicament as described herein may lead to a reduction in NAFLD activity score (NAS), steatosis score, lobular inflammation score, ballooning score, or fibrosis score, preferably NAS score, steatosis score, ballooning score or fibrosis score, more preferably fibrosis score. A reduction is preferably a reduction of at least one score level, alternatively at least two or three levels.

In some embodiments, any of the described effects are observed after at least 1 week, 8 days, 9 days, 10 days, 11 days, 12 days, 13 days, 2 weeks, 3 weeks, 4 weeks, 5 weeks, 6 weeks, 7 weeks, 8 weeks, 9 weeks, 10 weeks, 11 weeks, 12 weeks, 3 months, 4 months, 5 months, 6 months, a year or more of treatment, said treatment optionally involving an amount and an administration regimen as decribed elsewhere herein.

*Underlying diseases and conditions*

Diseases and conditions of the liver or symptoms thereof as described herein may be found in a large number of diseases and conditions which may be referred to as underlying diseases and conditions. It should therefore be understood that aspects of this disclosure useful for understanding the invention also encompass that the compositions provided herein may be used for treating, delaying, ameliorating, preventing or curing any underlying diseases and conditions.

For example, NAFLD is found in a large number of diseases and conditions which may be referred to as underlying diseases and conditions, as summarized in Table 1.

Table 1: Causes (underlying conditions and diseases) of NAFLD

| | |
|---|---|
| Obesity | Metabolic disorders of insulin resistance such as metabolic syndrome, syndrome X, insulin resistance syndrome, type 2 diabetes mellitus, Alström syndrome, Bardet-Biedl syndrome, lipodystrophy syndromes, PPAR$\gamma$ mutations<br>Leptin deficiency or resistance<br>Hypothalamic obesity<br>Prader-Willi syndrome |
| Genetic and metabolic diseases | Wilson disease, Weber-Christian disease, Wolman disease, cholesterol ester storage disease, abetalipoproteinemia, familial hypobetalipoproteinemia, Dorman-Chanarin syndrome, pseudoneonatal adrenoleukodystrophy, galactosemia, tyrosinemia, hereditary fructose intolerance, cystinuria, Sandhoff disease, pituitary dysfunction, hypothyroidism |
| Drugs and toxins | Corticosteroids, estrogens, NSAIDs, calcium antagonists, amiodarone, tamoxifen, tetracycline, chloroquine, perhexiline-maleate, antiretrovirals Industrial solvent dimethylformamide, paint thinners and solvents, petrochemical exposure, rapeseed cooking, cocaine |
| Extrahepatic conditions | Cardiac failure, inflammatory bowel disease and other intestinal diseases, intestinal bacterial overgrowth syndrome, cystic fibrosis, craniopharyngioma, pregnancy |
| Nutritional disorders | Jejunoileal bypass, gastric bypass, gastroplasty, biliopancreatic bypass, small bowel resection, total parenteral nutrition, prolonged starvation, rapid weight loss, protein malnutrition, high carbohydrate diet, Shwachman syndrome |
| Infective | Infection with hepatitis C, B, D virus |
| Other | Hepatic ischemia, age |
| NSAIDs, Nonsteroidal antiinflammatory drugs; PPAR, peroxisome proliferator-activated receptors. | |

Furthermore, portal hypertension may result from gastro-intestinal disorders, including primary sclerosing cholangitis (PSC) and chronic pancreatitis.

It should therefore be understood that aspects of this disclosure useful for understanding the invention also encompass that the compositions provided herein may be used for treating, delaying, ameliorating, preventing or curing any of the diseases or conditions mentioned in Table 1. Accordingly, aspects of the disclosure useful for understanding the invention encompass any of the compositions provided herein for use in treating, delaying, ameliorating, preventing or curing obesity, a genetic or metabolic disease, a drug or toxin induced condition, an extrahepatic condition, a nutritional disorder, a viral infection, hepatic ischemia, or senescence.

Accordingly, aspects of the disclosure useful for understanding the invention encompass any of the compositions provided herein for use in treating, delaying, ameliorating, preventing or curing metabolic disorders of insulin resistance (such as metabolic syndrome, syndrome X, insulin resistance syndrome, type 2 diabetes mellitus, Alström syndrome, Bardet-Biedl syndrome, lipodystrophy syndromes, PPARγ mutations), Leptin deficiency or resistance, Hypothalamic obesity or Prader-Willi syndrome. Aspdects of the disclosure useful for understanding the invention also encompass any of the compositions provided herein for use in treating, delaying, ameliorating, preventing or curing Wilson disease, Weber-Christian disease, Wolman disease, cholesterol ester storage disease, abetalipoproteinemia, familial hypobetalipoproteinemia, Dorman-Chanarin syndrome, pseudoneonatal adrenoleukodystrophy, galactosemia, tyrosinemia, hereditary fructose intolerance, cystinuria, Sandhoff disease, pituitary dysfunction or hypothyroidism. Aspects of the disclosure useful for understanding the invention also encompass any of the compositions provided herein for use in treating, delaying, ameliorating, preventing or curing toxicity or overdoses associated with corticosteroids, estrogens, NSAIDs, calcium antagonists, amiodarone, tamoxifen, tetracycline, chloroquine, perhexiline-maleate, antiretrovirals, Industrial solvent dimethylformamide, paint thinners and solvents, petrochemical exposure, rapeseed cooking or cocaine. Aspects of the disclosure useful for understanding the invention also encompass any of the compositions provided herein for use in treating, delaying, ameliorating, preventing or curing cardiac failure, inflammatory bowel disease and other intestinal diseases, intestinal bacterial overgrowth syndrome, cystic fibrosis, craniopharyngioma or pregnancy-related liver conditions. Aspects of the disclosure useful for understanding the invention also encompass any of the compositions provided herein for use in treating, delaying, ameliorating, preventing or curing liver-related conditions of Jejunoileal bypass, gastric bypass, gastroplasty, biliopancreatic bypass, small bowel resection, total parenteral nutrition, prolonged starvation, rapid weight loss, protein malnutrition, high carbohydrate diet or Shwachman syndrome. Aspects of the disclosure useful for understanding the invention also encompass any of the compositions provided herein for use in treating, delaying, ameliorating, preventing or curing hepatitis C, B, or D virus infections. Aspects of the disclosure useful for understanding the invention also encompass any of the compositions provided herein for use in treating, delaying, ameliorating, preventing or curing hepatic ischemia or senescence. Aspects of the disclosure useful for understanding the invention also encompass any of the compositions provided herein for use in treating, delaying, ameliorating, preventing or curing gastro-intestinal disorders, including Primary sclerosing cholangitis (PSC) and chronic pancreatitis.

In some aspects useful for understanding the invention, the compositions provided herein are for use in a method of managing liver symptoms in any of the above-mentioned conditions of Table 1. In some aspects useful for understanding the invention, the compositions provided herein are for use in a method of improving liver functioning in any of the above-mentioned conditions of Table 1. In some aspects useful for understanding the invention, the compositions provided herein are for use in delaying liver detoriation in any of the above-mentioned conditions of Table 1.

*Extrahepatic manifestations*

Extrahepatic manifestations may be understood to refer to extrahepatic disorders and/or symptoms that are a result of the diseases or conditions of the liver as described herein. It should therefore be understood that aspects of this disclosure useful for understanding the invention also encompasses that the compositions provided herein may be used for treating, delaying, ameliorating, preventing or curing any extrahepatic manifestations of a disease or condition of the liver as described herein.

Extrahepatic manifestations may be due to effects on organs, tissues or cells other than the liver. Extrahepatic manifestations may for example be due to effects on the brain, the vasculature or extravascular effects. In some aspects useful for understanding the invention, extrahepatic manifestations as described herein are may be hepatic encephalopathy, varices or ascites, preferably hepatic encephalopathy.

Without wishing to be bound by any particular theory, it is believed that the above-described compositions and their uses and methods may be based on the following mechanisms:

a) stimulating growth and/or activity of one or a limited number of beneficial bacteria in the intestinal tract,
b) inhibiting growth and/or activity of one or a limited number of pathogenic bacteria in the intestinal tract,
c) relatively increasing the attachment of non-pathogenic bacteria to the mucosa of the gastrointestinal surface,
d) reducing uncontrolled uptake of antigens, pro-inflammatory, bacteria or bacterial products by the gut,

e) providing anti-inflammatory activity at the intestinal surface,

f) increasing gut barrier functioning,

g) producing bacterial metabolites,

h) reducing the production of detrimental gut metabolites,

i) inducing wound repair,

or j) any combination of a) to i).

Without wishing to be bound by any particular theory, additionally or alternatively, the above-described compositions and their uses and methods may be based on the following liver-related mechanisms:

a) restoring hepatic insulin signaling,

b) reducing hepatic pro-fibrotic markers,

c) reducing hepatic lipid accumulation,

or d) any combination of a) to c).

Some particular aspects and embodiments of the disclosure are set out below.

In an aspect, this disclosure provides a composition that comprises bacterial members that are purified, wherein the bacterial members comprise *Faecalibacterium prausnitzii, Butyricoccus pullicaecorum, Roseburia inulinivorans, Akkermansia muciniphila, Lactiplantibacillus plantarum, Anaerostipes caccae,* and *Phocaeicola vulgatus,* and at least one of *Veillonella parvula, Veillonella atypica, Blautia obeum, Blautia wexlerae,* or *Blautia luti.* In some instances, the bacterial members can include *Veillonella parvula* or *Veillonella atypica.* In other instances, the bacterial members can include *Blautia obeum, Blautia wexlerae,* or *Blautia luti.* In yet other instances, the bacterial members can include:

- *Veillonella parvula* or *Veillonella atypica;* and
- *Blautia obeum, Blautia wexlerae,* or *Blautia luti.*

Advantageously, the composition of bacterial members may be useful for treating or preventing certain undesired conditions of the liver, for example, liver inflammation.

In some embodiments, the compositions can include certain strains of bacterial members. For example, in some instances, the bacterial members can include *Faecalibacterium prausnitzii* strain LMG P-29362. In some instances, the bacterial members can include *Butyricoccus pullicaecorum* strain LMG P-29360. In some instances, the bacterial members can include *Roseburia inulinivorans* strain LMG P-29365. In some instances, the bacterial members can include *Akkermansia muciniphila* strain LMG P-29361. In some instances, the bacterial members can include *Lactiplantibacillus plantarum* strain LMG P-29366. In some instances, the bacterial members can include *Anaerostipes caccae* strain LMG P-29359. In some instances, the bacterial members can include *Phocaeicola vulgatus* strain LMG17767. In some instances, the bacterial members can include *Veillonella parvula* strain DSM 2007. In some instances, the bacterial members can include *Blautia obeum* strain DSM 25238.

The composition can be formulated to provide a rapid and efficient therapeutic effect. In some instances, the composition can be formulated for delivery to the intestines. For example, the composition can be formulated as a powder, a suspension, a tablet, a food supplement, and the like. In some instances, for example, the bacterial members can be lyophilized. For example, the bacterial members may be freeze dried. In some instances, the composition of bacterial members can be provided in a suspension dosage form. The composition can include at least $10^5$ colony forming units of bacteria, for example, the composition can include between $10^5$ and $10^{11}$ colony forming units of bacteria. As described herein, the bacteria can be lyophilized or provided in an active culture format.

In a different aspect, this disclosure provides a method for improving a condition of the liver, the method including administering a composition including bacterial members to a subject in need thereof, wherein the bacterial members include *Faecalibacterium prausnitzii, Butyricoccus pullicaecorum, Roseburia inulinivorans, Akkermansia muciniphila, Lactiplantibacillus plantarum, Anaerostipes caccae,* and *Phocaeicola vulgatus,* and at least one of *Veillonella parvula, Veillonella atypica, Blautia obeum, Blautia wexlerae,* or *Blautia luti,* wherein administering the composition improves the condition of the liver, optionally as compared to the condition of the liver prior to administering the composition. In some instances, the bacterial members can include *Veillonella parvula* or *Veillonella atypica.* In some instances, the bacterial members can include *Blautia obeum, Blautia wexlerae,* or *Blautia luti.* In other instances, the bacterial members can include:

- *Veillonella parvula* or *Veillonella atypica;* and
- *Blautia obeum, Blautia wexlerae,* or *Blautia luti.*

The method can be useful for treating conditions of the liver. In aspects useful for understanding the invention, the condition can be, for example, any one of liver inflammation, obesity, a genetic or metabolic disease, a drug or toxin induced condition, an extrahepatic condition, a nutritional disorder, a viral infection, hepatic ischemia, or senescence. For example, in some instances the condition can include liver inflammation, and administering the composition can improved liver inflammation as evidenced by a reduction in the NAFLD activity score (NAS) of the subject. NAS is a methodology for feature-based scoring of histologic lesions of NAFLD which has been accepted in clinical and experimental settings. In some instances, for example, the NAS of the subject can be reduced by a numerical value of about 1.0 within 9 weeks of administering the composition, optionally as compared to a subject receiving a placebo. In some instances, the condition can include NASH. For example, the composition can be administered to a subject having NASH and can thereby delay the progression of NASH, optionally as compared to a subject inflicted with NASH that is administered a placebo instead of the composition.

In some instances, administering the composition to the subject can result in body weight gain, improved portal hypertension, or improved insulin sensitivity, optionally as compared to a subject with a comparable liver condition that is administered a placebo. In some instances, administering the composition can result in a reduction of about 40% in fasting blood insulin levels within two weeks of treatment, optionally as compared to a subject that is administered a placebo. In additional instances, administering the composition can result in a reduction of about 80% in intrahepatic vascular resistance (IHVR) within 10 weeks of treatment, optionally as compared to a subject administered a placebo. The composition can be administered according to any regime, for example, the composition can be administered as a single one-time dose, or the composition can be administered to the subject as routinely or periodically, for example, the composition can be administered to the subject once every 12 hours, 24 hours, or 48 hours for at least 7 days, 8 days, 9 days, 10 days, or more. In some instances, the composition is formulated for delivery to the intestines. In some instances, the bacterial members can be lyophilized. In some instances, the composition can be provided in a suspension dosage form. In some instances, the composition comprises *Faecalibacterium prausnitzii* strain LMG P-29362, *Butyricococcus pullicaecorum* strain LMG P-29360, *Roseburia inulinivorans* strain LMG P-29365, *Akkermansia muciniphila* strain LMG P-29361, *Lactiplantibacillus plantarum* strain LMG P-29366, *Anaerostipes caccae* strain LMG P-29359, *Phocaeicola vulgatus* strain LMG17767, *Veillonella parvula* strain DSM 2007, and *Blautia obeum* strain DSM 25238. The composition can include between $10^5$ and $10^{11}$ colony forming units of bacteria.

General information

Unless stated otherwise, all technical and scientific terms used herein have the same meaning as customarily and ordinarily understood by a person of ordinary skill in the art to which this invention belongs, and read in view of this disclosure.

*Sequence identity*

In the context of this disclosure, a nucleic acid molecule such as a nucleic acid molecule encoding a 16S rRNA gene is represented by a nucleic acid or nucleotide sequence which encodes a 16s rRNA.

It is to be understood that each nucleic acid molecule or protein fragment or polypeptide or peptide or derived peptide or construct as identified herein by a given sequence identity number (SEQ ID NO) is not limited to this specific sequence as disclosed. Each coding sequence as identified herein encodes a given protein fragment or polypeptide or peptide or derived peptide or construct or is itself a protein fragment or polypeptide or construct or peptide or derived peptide.

Throughout this application, each time one refers to a specific nucleotide sequence SEQ ID NO (take SEQ ID NO: X as example) encoding a given protein fragment or polypeptide or peptide or derived peptide or rRNA, one may replace it by:

    i. a nucleotide sequence comprising a nucleotide sequence that has at least 60%, 70%, 80%, 90%, 95% or 99% sequence identity with SEQ ID NO: X;
    ii. a nucleotide sequence the sequence of which differs from the sequence of a nucleic acid molecule of (i) due to the degeneracy of the genetic code; or
    iii. a nucleotide sequence that encodes an amino acid sequence that has at least 60%, 70%, 80%, 90%, 95% or 99% amino acid identity or similarity with an amino acid sequence encoded by a nucleotide sequence SEQ ID NO: X.

Another preferred level of sequence identity or similarity is 70%. Another preferred level of sequence identity or similarity is 80%. Another preferred level of sequence identity or similarity is 90%. Another preferred level of sequence identity or similarity is 95%. Another preferred level of sequence identity or similarity is 99%.

In the context of 16S rRNA genes, due to their high degree of conservation, preferred levels of sequence identity are generally higher. Thus, preferred levels of sequence identity for 16S rRNA genes are 97%, 97.5%, 98%, 98.5%, 99% or 99.5%.

Throughout this application, each time one refers to a specific amino acid sequence SEQ ID NO (take SEQ ID NO: Y as example), one may replace it by: a polypeptide represented by an amino acid sequence comprising a sequence that has at least 60%, 70%, 80%, 90%, 95% or 99% sequence identity or similarity with amino acid sequence SEQ ID NO: Y. Another preferred level of sequence identity or similarity is 70%. Another preferred level of sequence identity or similarity is 80%. Another preferred level of sequence identity or similarity is 90%. Another preferred level of sequence identity or similarity is 95%. Another preferred level of sequence identity or similarity is 99%.

Each nucleotide sequence or amino acid sequence described herein by virtue of its identity or similarity percentage with a given nucleotide sequence or amino acid sequence respectively has in a further preferred embodiment an identity or a similarity of at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% with the given nucleotide or amino acid sequence, respectively.

Each non-coding nucleotide sequence (i.e. of a promoter or of another regulatory region) could be replaced by a nucleotide sequence comprising a nucleotide sequence that has at least 60% sequence identity or similarity with a specific nucleotide sequence SEQ ID NO (take SEQ ID NO: A as example). A preferred nucleotide sequence has at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO: A. In a preferred embodiment, such non-coding nucleotide sequence such as a promoter exhibits or exerts at least an activity of such a non-coding nucleotide sequence such as an activity of a promoter as known to a person of skill in the art.

The terms "homology", "sequence identity" and the like are used interchangeably herein. Sequence identity is described herein as a relationship between two or more amino acid (polypeptide or protein) sequences or two or more nucleic acid (polynucleotide) sequences, as determined by comparing the sequences. In a preferred embodiment, sequence identity is calculated based on the full length of two given SEQ ID NO's or on a part thereof. Part thereof preferably means at least 50%, 60%, 70%, 80%, 90%, or 100% of both SEQ ID NO's. In the art, "identity" also refers to the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in Bioinformatics and the Cell: Modern Computational Approaches in Genomics, Proteomics and transcriptomics, Xia X., Springer International Publishing, New York, 2018; and Bioinformatics: Sequence and Genome Analysis, Mount D., Cold Spring Harbor Laboratory Press, New York, 2004.

"Sequence identity" and "sequence similarity" can be determined by alignment of two peptide or two nucleotide sequences using global or local alignment algorithms, depending on the length of the two sequences. Sequences of similar lengths are preferably aligned using a global alignment algorithm (e.g. Needleman-Wunsch) which aligns the sequences optimally over the entire length, while sequences of substantially different lengths are preferably aligned using a local alignment algorithm (e.g. Smith-Waterman). Sequences may then be referred to as "substantially identical" or "essentially similar" when they (when optimally aligned by for example the program EMBOSS needle or EMBOSS water using default parameters) share at least a certain minimal percentage of sequence identity (as described below).

A global alignment is suitably used to determine sequence identity when the two sequences have similar lengths. When sequences have a substantially different overall length, local alignments, such as those using the Smith-Waterman algorithm, are preferred. EMBOSS needle uses the Needleman-Wunsch global alignment algorithm to align two sequences over their entire length (full length), maximizing the number of matches and minimizing the number of gaps. EMBOSS water uses the Smith-Waterman local alignment algorithm. Generally, the EMBOSS needle and EMBOSS water default parameters are used, with a gap open penalty = 10 (nucleotide sequences) / 10 (proteins) and gap extension penalty = 0.5 (nucleotide sequences) / 0.5 (proteins). For nucleotide sequences the default scoring matrix used is DNAfull and for proteins the default scoring matrix is Blosum62 (Henikoff & Henikoff, 1992, PNAS 89, 915-919).

Alternatively percentage similarity or identity may be determined by searching against public databases, using algorithms such as FASTA, BLAST, etc. Thus, the nucleic acid and protein sequences of some embodiments of the present disclosure can further be used as a "query sequence" to perform a search against public databases to, for example, identify other family members or related sequences. Such searches can be performed using the BLASTn and BLASTx programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to oxidoreductase nucleic acid molecules of the disclosure. BLAST protein searches can be performed with the BLASTx program, score = 50, wordlength = 3 to obtain amino acid sequences homologous to protein molecules of the disclosure. To obtain gapped alignments for comparison purposes, Gapped BLAST can be utilized as described in Altschul et al., (1997) Nucleic Acids Res. 25(17): 3389-3402. When utilizing BLAST and Gapped BLAST programs, the default parameters of the respective programs (e.g., BLASTx and BLASTn) can be used. See the homepage of the National Center for Biotechnology Information accessible on the world wide web at www.ncbi.nlm.nih.gov/.

Optionally, in determining the degree of amino acid similarity, the skilled person may also take into account so-called conservative amino acid substitutions. As used herein, "conservative" amino acid substitutions refer to the interchangeability of residues having similar side chains. Examples of classes of amino acid residues for conservative substitutions are given in the Tables below.

| Acidic Residues | Asp (D) and Glu (E) |
|---|---|
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

Alternative conservative amino acid residue substitution classes :

| 1 | A | S | T |
|---|---|---|---|
| 2 | D | E | |
| 3 | N | Q | |
| 4 | R | K | |
| 5 | I | L | M |
| 6 | F | Y | W |

Alternative physical and functional classifications of amino acid residues:

| Alcohol group-containing residues | S and T |
|---|---|
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P and T |
| Flexible residues | Q, T, K, S, G, P, D, E, and R |

For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to Ser; Arg to Lys; Asn to Gln or His; Asp to Glu; Cys to Ser or Ala; Gln to Asn; Glu to Asp; Gly to Pro; His to Asn or Gln; Ile to Leu or Val; Leu to Ile or Val; Lys to Arg; Gln or Glu; Met to Leu or Ile; Phe to Met, Leu or Tyr; Ser to Thr; Thr to Ser; Trp to Tyr; Tyr to Trp or Phe; and, Val to Ile or Leu.

*Gene or coding sequence*

The term "gene" means a DNA fragment comprising a region (transcribed region), which is transcribed into an RNA molecule (e.g. an mRNA) in a cell, operably linked to suitable regulatory regions (e.g. a promoter). A gene will usually comprise several operably linked fragments, such as a promoter, a 5' leader sequence, a coding region and a 3'-nontranslated sequence (3'-end) e.g. comprising a polyadenylation- and/or transcription termination site. A chimeric or recombinant gene is a gene not normally found in nature, such as a gene in which for example the promoter is not associated in nature with part or all of the transcribed DNA region. "Expression of a gene" refers to the process wherein a DNA region which is operably linked to appropriate regulatory regions, particularly a promoter, is transcribed into an RNA, which is biologically active, i.e. which is capable of being translated into a biologically active protein or peptide.

*Proteins and amino acids*

The terms "protein" or "polypeptide" or "amino acid sequence" are used interchangeably and refer to molecules consisting of a chain of amino acids, without reference to a specific mode of action, size, 3-dimensional structure or origin. In amino acid sequences as described herein, amino acids or "residues" are denoted by three-letter symbols. These three-letter symbols as well as the corresponding one-letter symbols are well known to a person of skill in the art and have the following meaning: A (Ala) is alanine, C (Cys) is cysteine, D (Asp) is aspartic acid, E (Glu) is glutamic acid, F (Phe) is phenylalanine, G (Gly) is glycine, H (His) is histidine, I (Ile) is isoleucine, K (Lys) is lysine, L (Leu) is leucine, M (Met) is methionine, N (Asn) is asparagine, P (Pro) is proline, Q (Gln) is glutamine, R (Arg) is arginine, S (Ser) is serine, T (Thr) is threonine, V (Val) is valine, W (Trp) is tryptophan, Y (Tyr) is tyrosine. A residue may be any proteinogenic amino acid, but also any non-proteinogenic amino acid such as D-amino acids and modified amino acids formed by post-translational modifications, and also any non-natural amino acid.

In this document and in its claims, the verb "to comprise" and its conjugations is used in its non-limiting sense to mean that items following the word are included, but items not specifically mentioned are not excluded. In addition, the verb "to consist" may be replaced by "to consist essentially of" meaning that a composition as described herein may comprise additional component(s) than the ones specifically identified, said additional component(s) not altering the unique characteristic of this disclosure. In addition, the verb "to consist" may be replaced by "to consist essentially of" meaning that a method as described herein may comprise additional step(s) than the ones specifically identified, said additional step(s) not altering the unique characteristic of this disclosure.

Reference to an element by the indefinite article "a" or "an" does not exclude the possibility that more than one of the element is present, unless the context clearly requires that there be one and only one of the elements. The indefinite article "a" or "an" thus usually means "at least one".

As used herein, with "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments described herein are capable of operation in other sequences than described or illustrated herein.

The word "about" or "approximately" when used in association with a numerical value (e.g. about 10) preferably means that the value may be the given value (of 10) more or less 1% of the value.

As used herein, the term "and/or" indicates that one or more of the stated cases may occur, alone or in combination with at least one of the stated cases, up to with all of the stated cases.

Various embodiments are described herein. Each embodiment as identified herein may be combined together unless otherwise indicated. Titles, subtitles, headings and the likes are used herein solely for ease of reading and are not intended to limit or restrict the disclosure in any way.

One skilled in the art will recognize many methods and materials similar or equivalent to those described herein, which could be used in the practice of the present invention. Indeed, the present invention is defined by the appended claims and is in no way limited to the methods and materials described. It is also understood that the disclosure encompasses the generalization of aspects of the following examples to the preceding disclosure.

The present invention is further described by the following examples which should not be construed as limiting the scope of the invention, which is defined by the appended claims.

Description of the figures

Figure 1. Experimental design. HFGFD-V: group of rats receiving sham gavage (vehicle); HFGFD-C: group of rats receiving the bacterial consortium daily; HFGFD-Tr: group of rats receiving intestinal microbiota (IM) collected from control lean rats (1x transplantation followed by sham gavage). Abbreviations: HFGFD, high-fat, high-glucose/fructose diet.

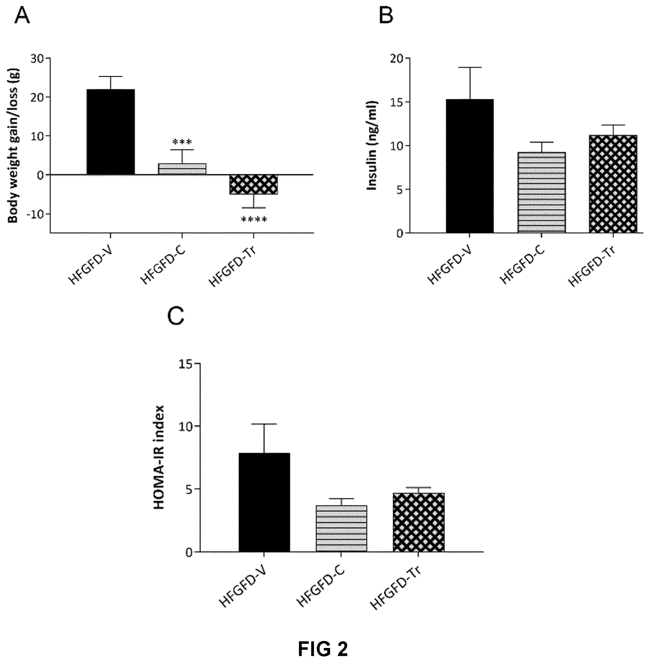

Figure 2. Body weight gain and evaluation of insulin resistance. (A) Body weight gain after 2 weeks of intervention. (B) Fasting insulin levels measured in the blood after 2 weeks of intervention. (C) Calculated Homeostatic Model Assessment for Insulin Resistance index. Abbreviations: HFGFD, high-fat, high-glucose/fructose diet.

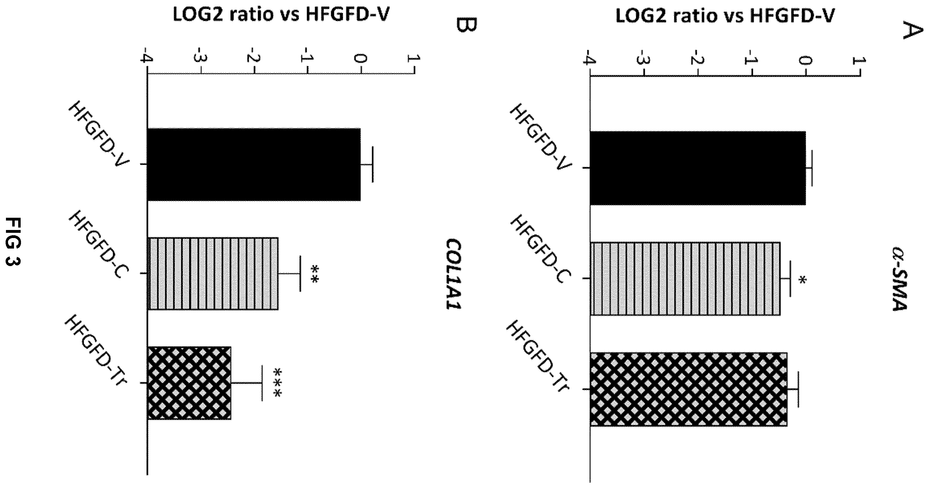

Figure 3. Relative mRNA expression of (A) $\alpha$-SMA and (B) COL1A1 by quantitative RT-PCR, expressed as a log2 ratio. beta-Actin was used as an endogenous control, and results were normalized to the HFGFD-V group. Abbreviations: $\alpha$-SMA, alpha-Smooth muscle actin; COL1A1, Collagen type I alpha 1 chain; HFGFD, high-fat, high-glucose/fructose diet.

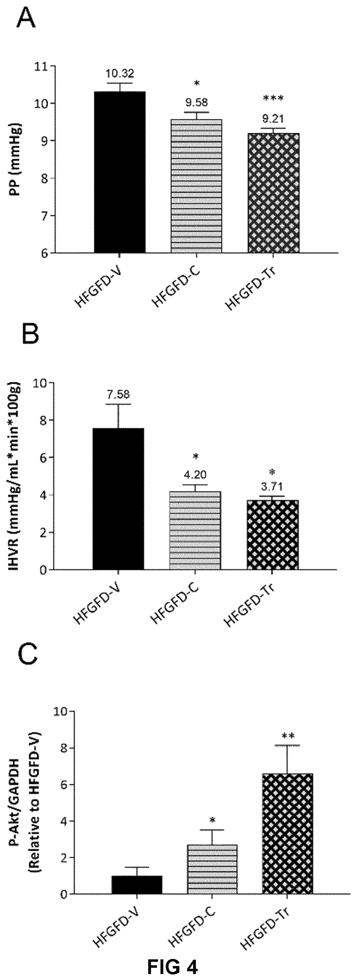

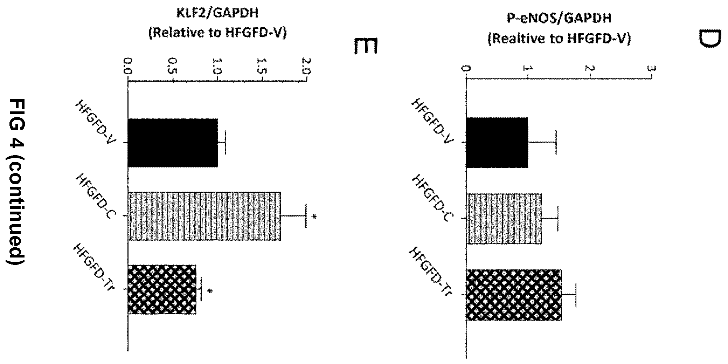

Figure 4. Liver hemodynamic parameters and intrahepatic markers of endothelial dysfunction. (A) Portal pressure (PP). (B) Intrahepatic vascular resistance (IHVR). (C-E) Western blot analysis of intrahepatic markers of endothelial dysfunction. Bar diagrams show the quantification of phosphorylated (P)-Akt, P-eNOS, and KLF2 using glyceraldehyde 3-phosphate dehydrogenase (GAPDH) as a loading control and normalized to the HFGFD-V group. Abbreviations: Akt, Protein kinase B; eNOS, Endothelial nitric oxide synthase; HFGFD, high-fat, high-glucose/fructose diet; KLF2, Kruppel Like Factor 2.

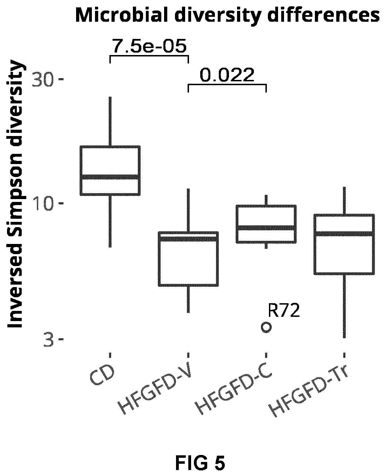

Figure 5. Inverse Simpson diversity index. Numbers indicate t-test p-values, computed without the outlier R72.

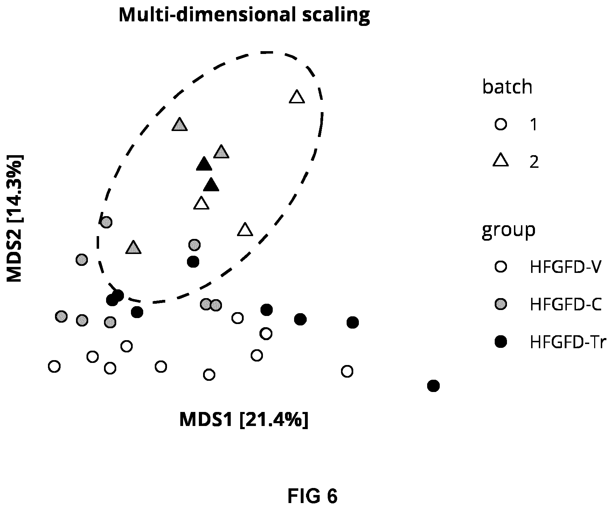

Figure 6. Multi-dimensional scaling (MDS). MDS is based on Bray-Curtis distances.

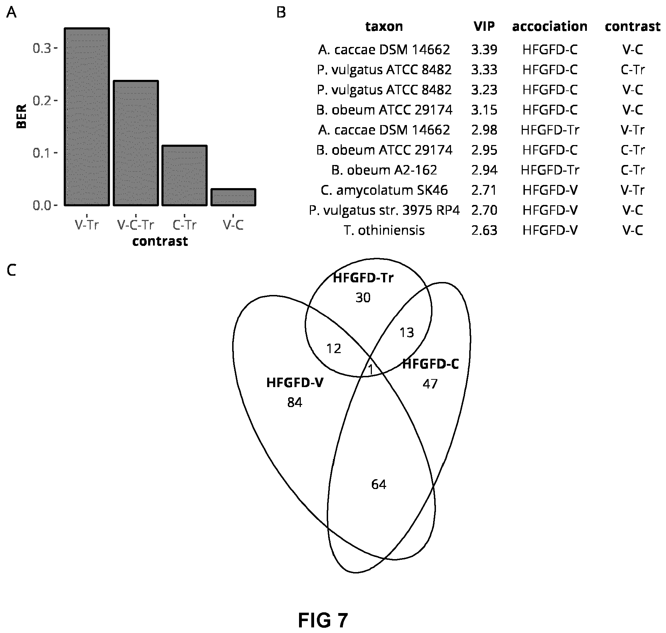

Figure 7. sPLS-DA classification. (A) per contrast BER. (B) top 10 discriminative taxa. (C) Euler plot of contrast specific taxa. Discriminant taxa: strain identifiers correspond with hits documented by the WMS taxonomic composition analysis performed by CosmosID and reflect the composition of the database used for the analysis. These should be considered as proxies for strains actually present in each of the treatment groups. Euler plot - numbers should be interpreted as follows: for HFGFD-C, 47 induced taxa were discriminant for distinction against HFGFD-V or HFGFD-Tr, or both. 13 induced taxa were discriminant against HFGFD-V in both the V-Tr and V-C contrasts. 12 HFGFD-V and HFGFD-Tr associated taxa were suppressed by HFGFD-C. 91 HFGFD-V associated taxa were suppressed by HFGFD-C or HFGFD-Tr, or both.

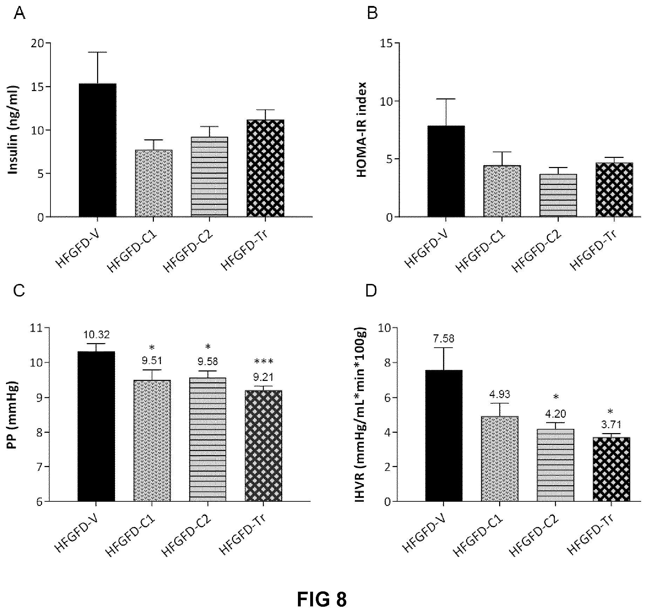

Figure 8. Effects of 6- and 9-strain consortia on (A) fasting blood insulin levels, (B) HOMA-IR index and (C-D) Liver hemodynamics. HFGFD-V: group of rats receiving sham gavage (vehicle); HFGFD-C1: group of rats receiving the 6-strain consortium daily; HFGFD-C2: group of rats receiving the 9-strain consortium daily; HFGFD-Tr: group of rats receiving intestinal microbiota (IM) collected from control rats (1x transplantation followed by sham gavage).

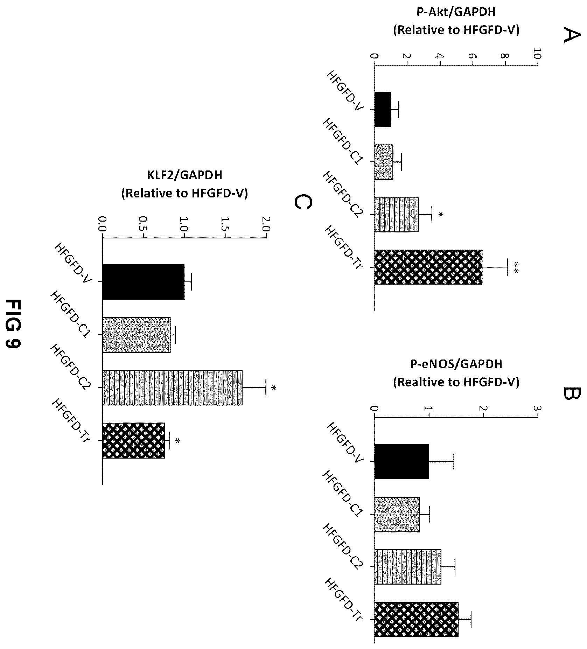

Figure 9. Effects of 6- and 9-strain consortia on endothelial dysfunction markers. (A) Hepatic AKT phosphorylation, (B) eNOS phosphorylation, and (C) KLF2. HFGFD-V: group of rats receiving sham gavage (vehicle); HFGFD-C1: group of rats receiving the 6-strain consortium daily; HFGFD-C2: group of rats receiving the 9-strain consortium daily; HFGFD-Tr: group of rats receiving intestinal microbiota (IM) collected from control rats (1x transplantation followed by sham gavage). Legend: AKT, AKT Serine/Threonine Kinase; eNOS, Endothelial nitric oxide synthase; KLF2, Kruppel-like factor 2.

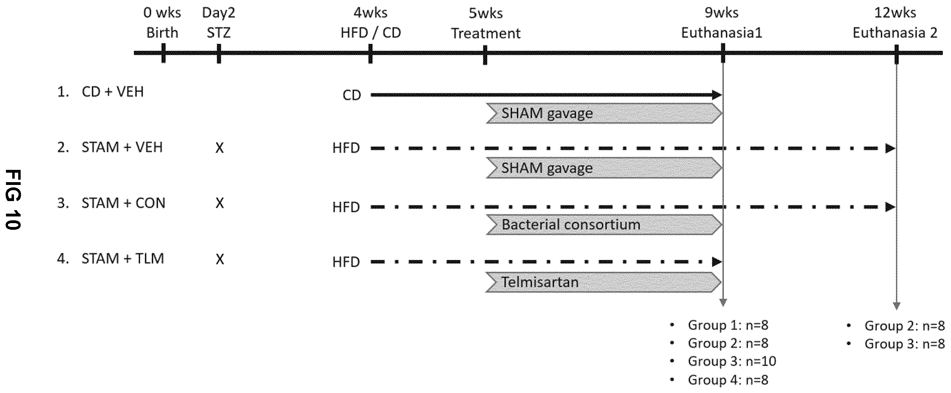

Figure 10. STAM™ study design. C57BL/6J male mice were subcutaneously injected with 200 μg STZ, a β-cell toxin, two days after birth. At 4 weeks of age animals received high-fat diet (HFD) or regular control diet (CD). At 5 weeks of age treatment was initiated: control diet animals and STAM mice received sham gavage (CD+VEH and STAM+VEH, respectively) for a total of 4 weeks. In the two test groups, STAM animals received either the 9-strain consortium ($10^9$ CFU/d) (STAM+CON) or Telmisartan (10 mg/kg/d) (STAM+TLM) also for a total of 4 weeks. At 9 weeks of age animals were sacrificed (euthanasia 1). Two groups of STAM animals belonging to the vehicle control or the consortium groups were followed for an additional three weeks until sacrifice at 12 weeks of age (euthanasia 2).

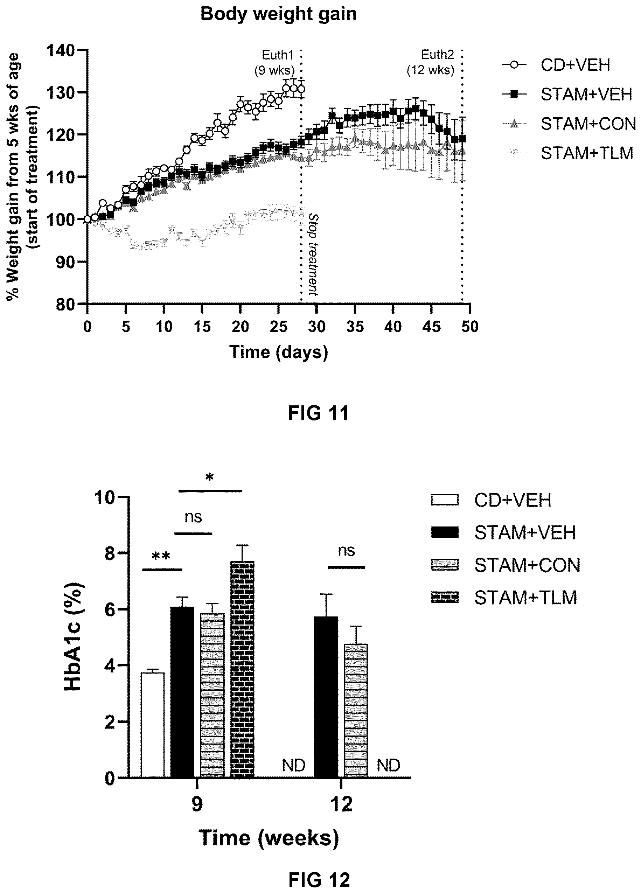

Figure 11. 9-strain consortium improves body weight gain at late stages of disease in STAM™ mice. Body weight gain is shown relatively to the weight recorded at 5 weeks of age, when the 9-strain consortium and Telmisartan (TLM) treatment initiated (i.e., one week after HFD started). Animals were divided in several groups and sacrificed at different moments in time (i.e., 7, 9, and 12 weeks of age, to target steatosis/steatohepatitis, early-stage fibrosis, and late-stage fibrosis, respectively). Treatment with either TLM or the 9-strain consortium initiated at 5 weeks of age and were given for a period of 2 weeks (until euthanasia 1) or for a period of 4 weeks (until euthanasia 2). Some animals within the 9-strain consortium- and vehicle-treated groups were followed up to 12 weeks of age (euthanasia 3). Legend: CD, Control diet group; Euth, Euthanasia; TLM, Telmisartan; VEH, Vehicle; CON, 9-strain consortium.

**Figure 12. 9-strain consortium improves whole blood HbA1c. Whole blood HbA1c (%) measured in the STAM™ study.** Whole blood was collected at 9 and 12 weeks of age (euthanasia 1 and 2, respectively) in heparinized tubes and used for analysis of glycated hemoglobin, a marker that indicates the presence of excessive glucose in the bloodstream. Legend: CD, Control diet group; TLM, Telmisartan; VEH, Vehicle; CON, 9-strain consortium.

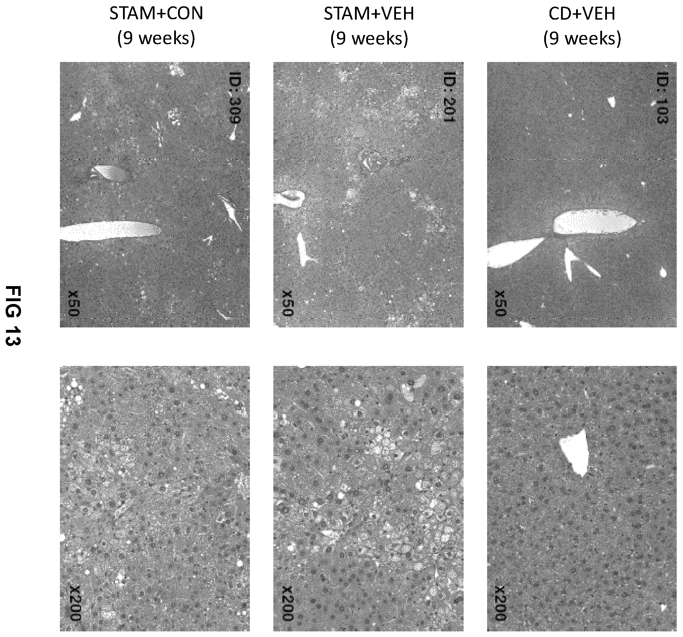

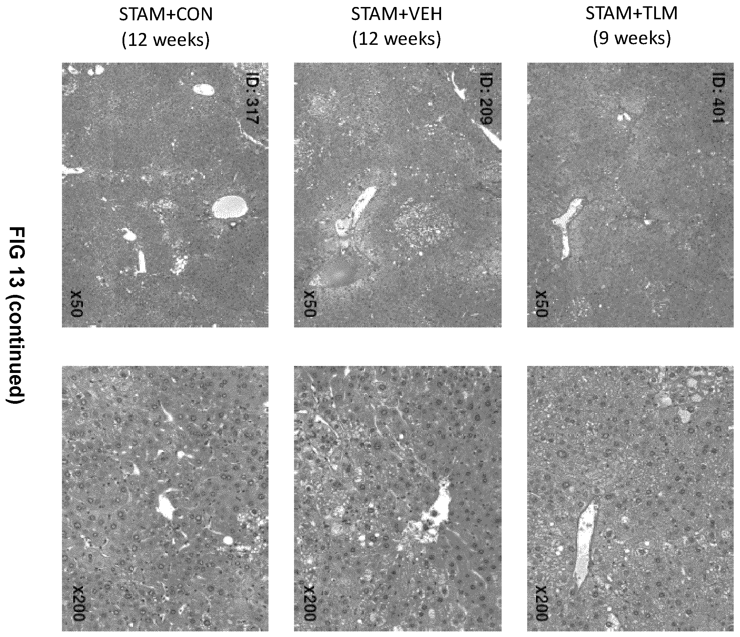

**Figure 13. Representative images of H&E-stained liver sections collected from the STAM™ study.** Liver sections were collected at both 9 and 12 weeks of age (euthanasia 1 and 2, respectively).

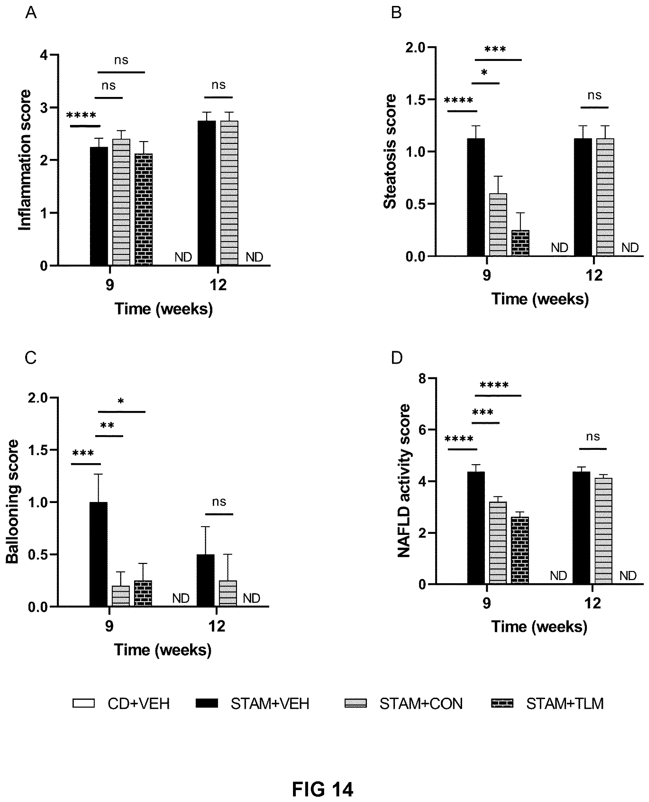

**Figure 14. NAFLD activity score (NAS) in the STAM™ study.** Histopathology was evaluated in H&E stained liver sections, and the NAS score was calculated according to the criteria of Kleiner.(22) The components (A) inflammation, (B) steatosis, (C) ballooning, and (D) the composite score were evaluated at both 9 and 12 weeks of age (euthanasia 1 and 2, respectively). ND: not determined for the CD+VEH and STAM+TLM groups at 12 weeks.

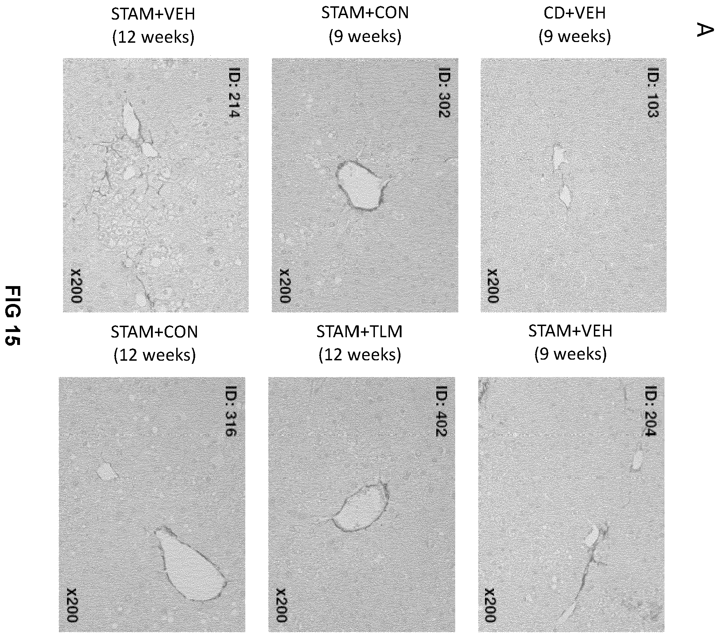

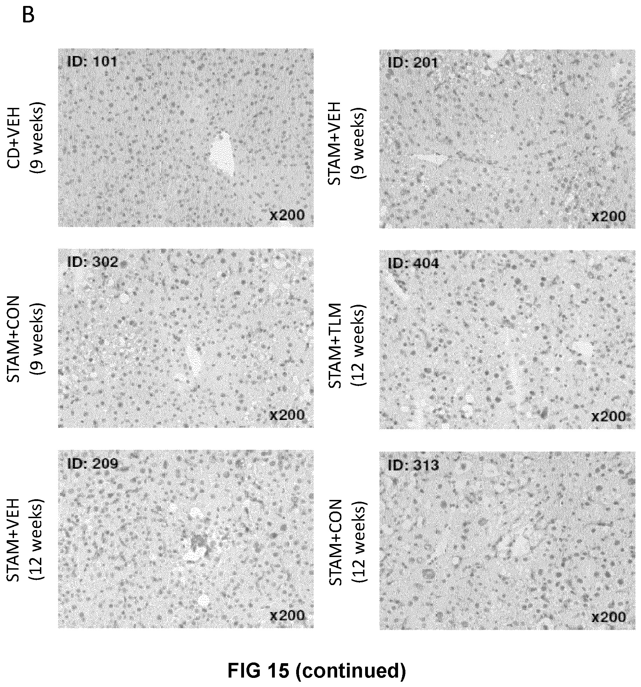

**Figure 15. Representative images of (A) picro-Sirius red-stained, and (B) F4/80 immunostained liver sections collected from the STAM™ study.** Liver sections were collected at both 9 and 12 weeks of age (euthanasia 1 and 2, respectively).

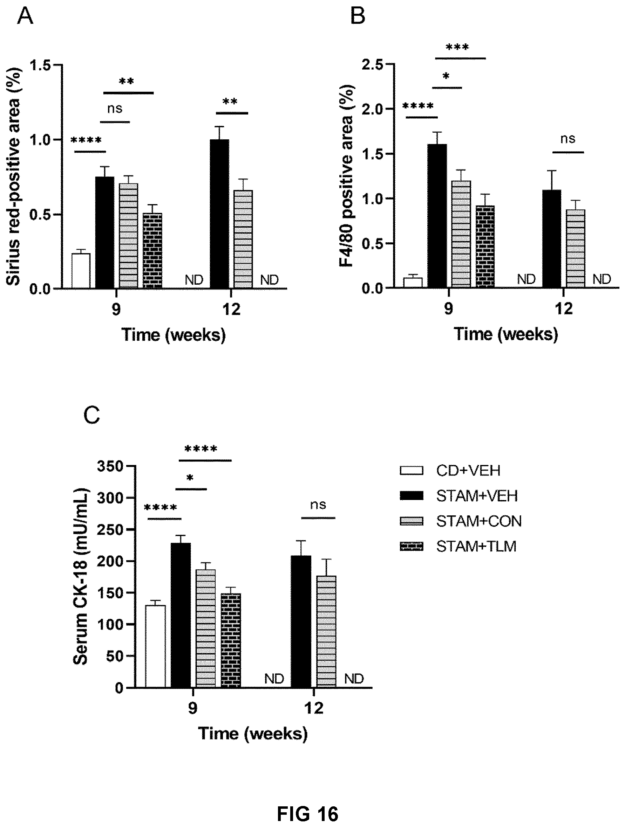

**Figure 16. Hepatic fibrosis area, F4/80 positive area, and serum CK-18 levels in the STAM™ study.** (A) Fibrosis assessed in Sirius red-stained liver sections. (B) Immunohistochemistry of macrophages evaluated in F4/80-immunostained sections. (C) Serum levels of CK-18, a marker for apoptotic hepatocytes. All markers were evaluated at both 9 and 12 weeks of age (euthanasia 1 and 2, respectively). ND: not determined for the CD+VEH and STAM+TLM groups at 12 weeks.

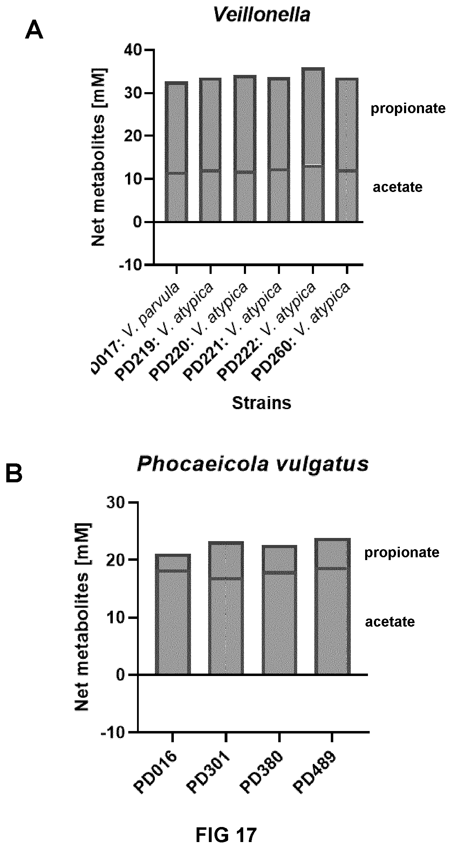

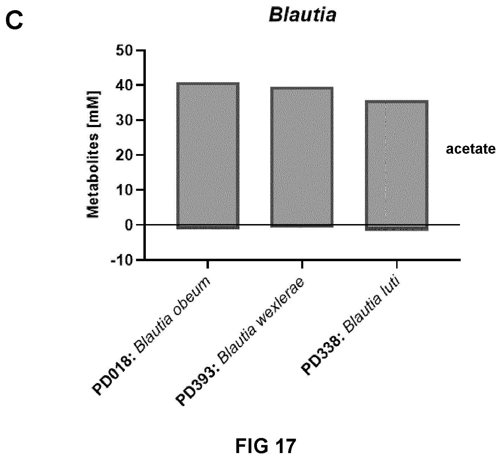

**Figure 17. Short chain fatty acid (SCFA) profiles in HV5 medium.** (A) *Veillonella parvula* and *Veillonella atypica* strains. (B) *Phocaeicola vulgatus* strains. (C) *Blautia obeum, Blautia wexlerae,* and *Blautia luti* strains.

## Examples

In Example 1, the effectiveness of a bacterial consortium composed of nine human gut commensal strains was tested in a relevant *in vivo* animal disease model, i.e. the rat NASH model of portal hypertension (PHT). Results were compared to animals receiving one-time fecal transplant from lean rats. The bacterial consortium was shown to improved body weight gain and portal hypertension; moreover, endothelial dysfunction markers also improved, due to restored insulin sensitivity. In addition, liver transcriptomics identified numerous pro-fibrotic pathways being favorably modulated.

In Example 2, the 9-strain consortium of example 1 was compared with a 6-strain consortium in the rat NASH model. The 9-strain consortium includes the 6 strains of the 6-strain consortium, plus 3 additional species that were included to increment propionate production. Both consortia were effective: the 9-strain consortium showed consistent results across the multiple endpoints, and the 6-strain consortium induced protective effects on several endpoints including fasting insulin levels, HOMA-IR index, and liver hemodynamics (PP, IHVR).

In Example 3, the effectiveness of the 9-strain consortium was additionally tested in another relevant *in vivo* animal disease model, i.e. the STAM™ mouse model of NASH. Administration of the 9-strain consortium confirmed its anti-fibrotic effects by 12 weeks of age. In addition, F4/80 immunohistochemistry was concomitant with reduced hepatic inflammation, and serum levels of cytokeratin-18 (CK-18), a blood marker of apoptotic hepatocytes that correlates with disease severity was significantly reduced 5 weeks after treatment.

Altogether these results show that microbial-based approaches can be used for the management of liver conditions such as NAFLD, and that consortia of gut commensals offer patients a safe, convenient and long-term solution to delay disease progression.

The 6-strain consortium tested comprises the following bacterial species:

- *Faecalibacterium prausnitzii* (strain LMG P-29362)
- *Butyricococcus pullicaecorum* (strain LMG P-29360)
- *Roseburia inulinivorans* (strain LMG P-29365)
- *Akkermansia muciniphila* (strain LMG P-29361)
- *Lactiplantibacillus plantarum* (strain LMG P-29366)
- *Anaerostipes caccae* (strain LMG P-29359)

The 9-strain consortium tested comprises the following bacterial species:

- *Faecalibacterium prausnitzii* (strain LMG P-29362)
- *Butyricococcus pullicaecorum* (strain LMG P-29360)
- *Roseburia inulinivorans* (strain LMG P-29365)
- *Akkermansia muciniphila* (strain LMG P-29361)
- *Lactiplantibacillus* plantarum (strain LMG P-29366)
- *Anaerostipes caccae* (strain LMG P-29359)

- *Phocaeicola vulgatus* (strain LMG17767)
- *Veillonella parvula* (strain DSM 2007)
- *Blautia obeum* (strain DSM 25238)

A 6-strain consortium has also been described in WO2017/134240. It was shown in WO2017/134/240 that the consortium was robust, independent of the exact strain that was included for each species. Thus, the skilled person understands that the results presented here can be obtained using other than the exemplified strains.

Example 1: Evaluation of a 6-strain consortium in the rat NASH model of portal hypertension (PHT)

Materials and methods

*Experimental design*

For model development, Sprague-Dawley rats were fed HFGFD (high-fat diet and a high-glucose/fructose syrup) for 8 weeks (Figure 1). After that, they were randomized into three groups: HFGFD-vehicle (HFGFD-V, n = 13), HFGFD-consortium of nine human commensal bacterial strains (HFGFD-C, n = 11) and HFGFD-transplanted with fecal matter from lean rats (HFGFD-Tr, n = 11). HFGFD-V individuals received oral probing vehicle (sterile PBS), while individuals in HFGFD-C and HFGFD-Tr groups were subjected to different microbiota-based treatments, which consisted of oral administration of bacterial commensals and intestinal microbiota (IM) transplantation, respectively. Each treatment is extensively described in the following section. The treatment period lasted 2 weeks during which animals were maintained on the original diet (Figure 1). At week 10, liver hemodynamics and blood biochemistry were performed, and liver tissue samples were collected for histological and molecular analyses. In addition, the cecum content was collected for shotgun sequencing of gut microbes.

*Animal model and diet*

Male Sprague-Dawley OFA rats (Charles River Laboratories, L'Arbresle, France), weighting 200-220 g at the beginning of the experiments, were used for the diet-induced NASH model, as previously described in (9). Rats were housed under a 12-hour light/dark cycle at constant temperature ($24 \pm 1°C$) and relative humidity ($55 \pm 10\%$). Animals had *ad libitum* access to a high-fat high-glucose/fructose diet (HFGFD) consisting of 30% fat (butter, coconut oil, palm oil, beef tallow) with mainly saturated fatty acids (5.73 kcal/g; Ssniff Spezialdiäten GmbH, Soest, Germany), supplemented with cholesterol (1 g/kg) and a beverage of high glucose/fructose content (42 g/L, 45% glucose and 55% fructose) providing 157.7 kcal/L. Body weight and food and drink consumption were monitored once a week.

All procedures were conducted in accordance with European Union Guidelines for Ethical Care of Experimental Animals (EC Directive 86/609/EEC for animal experiments), approved by the Animal Care Committee of the Vall d'Hebron Institut de Recerca (Barcelona, Spain), and conducted in the animal facilities of Vall d'Hebron Institut de Recerca.

*Bacterial consortium treatment*

The consortium treatment started after 8 weeks of diet intervention. Freeze-dried vials containing the nine bacterial strains were resuspended under anaerobic conditions ($N_2:CO_2:H_2$: 80:10:10). After decontamination of the anaerobic chamber and the lid of the vials, the concentrated product strains were gently resuspended in previously filtered 20 ml anaerobic DPBS (Dulbecco's phosphate buffered saline without calcium chloride and magnesium chloride, Sigma Aldrich). The content of the vial was divided in sterile 50 ml-falcon tubes by pouring and taken out of the anaerobic chamber. The 50 ml-falcons were centrifuged at 5340*g* at room temperature for 10 minutes. Falcons were brought back to the anaerobic chamber and the supernatant was then discarded by pouring. Each pellet was resuspended in 3 ml of anaerobic DPBS and then 3.5 ml of DPBS were added in each tube to obtain a final volume of 6.5 ml per vial. All the sample was finally transferred to a glass, closed sterile vial with anaerobic atmosphere. Once the consortium was reconstituted, 1 ml of suspension was immediately administered per individual (corresponding to a daily dose of $2x10^9$ CFU), using a disposable oral probe (Biochrom Ltd, Cambridge, UK) and repeating the procedure every 24 hours for 10 consecutive days. For individuals belonging to the vehicle group, the same procedure was followed but sterile DPBS was administered instead. Rats receiving the bacterial consortium were housed individually throughout the treatment period to avoid cross-contamination.

*Intestinal microbiota (IM) transplantation*

The IM transplant was performed after 8 weeks of diet intervention. To achieve higher homogeneity in the transplantation procedure, feces from three control (lean) rat donors were pooled to be used for the IM transplantation. With the aim of reducing gastric acidity and thus increasing the survival of the microorganisms, omeprazole was administered orally at a dose of 50 kg/day during the 3 days prior to intestinal decontamination. To proceed with intestinal emptying, rats were maintained isolated in fast grills, and two oral doses of CitraFleet (sodium picosulfate, 0.16 mg/ml and magnesium oxide 51.2 mg/ml) of 1 ml and 2 ml were administered 24h and 12h, respectively, before the transplant. These administrations were accompanied by 2 ml of water each. Recolonization was performed by a single oral gavage, for which 100 mg of the fecal pool were dissolved in 2 ml of sterile DPBS.

*Blood biochemistry*

Blood samples were collected from the cava vein at sacrifice. Glucose, bilirubin, alanine aminotransferase (ALT), aspartate aminotransferase (AST), cholesterol, high density lipoprotein (HDL), low density lipoprotein (LDL), triglycerides and albumin were measured with standard methods (see Garcia-Lezana, T et al. Restoration of a Healthy Intestinal Microbiota Normalizes Portal Hypertension in a Rat Model of Nonalcoholic Steatohepatitis. HEPATOLOGY 2018;67(4)). Insulin was measured using an ELISA kit following the manufacturer's instructions (EMD Millipore, Billerica, MA, United States). Insulin resistance was estimated applying the homeostasis model of insulin resistance index (HOMA-IR): (fasting insulin (ng/mL) * fasting glucose (mg/dL))/405.

*Histological analysis*

For histological analyses, liver samples were extracted, fixed in 4% formalin, embedded in liquid paraffin at 65°C and sectioned in $4\mu m$ thick slides. Samples were then stained with hematoxylin and eosin (H&E) to assess liver parenchyma and with Sirius Red to detect collagen fibers. All stained liver samples were examined by an expert liver pathologist blinded to the interventions performed on the animals.

The diagnosis of NASH was established based on the presence of all three characteristic patterns of the disease, which include the coexistence of steatosis, lobular inflammation, and hepatocellular ballooning. The NASH-CRN Activity Score (NAS) was used to quantify NASH activity. NAS comprises the unweighted sum of the histological components: steatosis (0-3), lobular inflammation (0-3), and hepatocellular ballooning (0-2). Fibrosis was also classified in five stages according to the NASH-CRN system, ranging from F0 (no fibrosis) to F4 (cirrhosis).

*Liver hemodynamics*

The hemodynamic measurements were performed in fasted rats under intraperitoneal anesthesia with ketamine (100 mg/Kg) plus midazolam (5 mg/kg) and body temperature maintained at 37°C. Mean arterial pressure (MAP) was measured by catheterization of the femoral artery and portal pressure (PP) assessed by ileocolic vein catheterization using highly sensitive pressure transducers (Harvard apparatus, Holliston, MA, USA). Superior mesenteric artery (SMA) blood flow (SMABF, mL/ [min*100g]) and portal blood flow (PBF, mL/[min*100g]) were measured with a perivascular ultrasonic transit-time flow probe (1 mm diameter, Transonic systems Inc, Ithaca, NY, USA). SMA resistance (SMAR) and intrahepatic vascular resistance (IHVR, mmHg/mL*min*100g) were calculated as ([MAP-PP]/SMABF) and (PP/PBF), respectively.

*Western blot*

Livers were perfused with saline for exsanguination and samples were directly frozen in liquid nitrogen and stored at -80°C until further use. Then, liver samples were crushed cold, avoiding defrosting, until turning them manually into dust. They were then homogenized in Triton-lysis buffer, sonicated, and centrifuged at 14,000rpm for 10min at 4°C. Supernatant total protein concentration was quantified by BCA protein assay kit (Thermo Fisher scientific, Waltham, MA, USA). Forty (40) $\mu g$ of protein were run on a 10% sodium dodecyl sulphate-polyacrylamide gel electrophoresis (SDS-PAGE). Proteins separated by SDS-PAGE were transferred onto a polyvinylidene fluoride (PVDF) membrane (Thermo Fisher scientific, Waltham, MA, USA). Membranes were washed with TTBS 1X several times and blocked for 1h at RT with Phospho-BLOCKER™ 5% (Cell biolabs, San Diego, CA, USA) to detect the phosphorylated proteins P-Akt (1/500, Cell signaling, Danvers, MA, USA) and P-eNOS (Ser1177, 1/250) or with skimmed milk to detect Kruppel Like Factor 2 (KLF2) (1/200, Santa Cruz biotechnology, Dallas, TX, USA). GAPDH antibody (1/5000, Ambion, Austin, TX, USA) was used as loading control. Membranes were developed using ECL kit (GE Healthcare; Little Chalfont, UK) and protein expression was finally determined by densitometry analysis bands using Image Studio Lite (Lincoln, NE, USA).

*Real-time PCR*

Liver and ileum samples were maintained at least 24h in RNA*later* (Thermo Fisher scientific, Waltham, MA, USA) and then stored at -80°C until further analysis. Total RNA was extracted by using the RNeasy mini Kit (QIAGEN, Venlo, Nederland) and retro-transcribed to complementary DNA (High capacity cDNA reverse transcription, Thermo Fisher Scientific, Waltham, MA, USA). Twenty (20) ng of cDNA was added to Taqman universal PCR master mix plus the specific Taqman probes for $\alpha$-SMA (alpha-Smooth muscle actin) and COL1A1 (Collagen type I alpha 1 chain) (Life Technologies Ltd, Renfrew, UK) and loaded in 384-well plates (Thermo Fisher scientific, Waltham, MA, USA). Quantitative reverse-transcription polymerase chain reaction (qRT-PCR) was performed using 7900HT Fast Real-Time PCR system (Thermo Fisher scientific, Waltham, MA, USA). The relative gene expression was normalized to beta-Actin. Data was analyzed using the Relative Quantification qPCR Application in Thermofisher Cloud.

*Caecal whole metagenome shotgun*

Genomic DNA was extracted from approx. 0.2 g of caecum content of each rat using the ZymobioMics DNA Miniprep Kit (Zymo Research, Irvine, CA, USA) according to manufacturer's instructions and quantified using the Qubit™ Flex Fluorometer (Thermo Fisher Scientific, Wilmington, DE, USA) and used for whole metagenome shotgun sequencing. Library preparation and sequencing were performed by CosmosID (Rockville, MD, USA) to generate 2x150 bp Illumina reads. Unassembled sequencing reads (≥12M read depth) were analyzed using the CosmosID bioinformatics platform for taxonomic and functional profile profiling.

Whole metagenome shotgun (WMS) sequencing, taxonomic- and pathway profiling was performed by CosmosID (Rockville, MD, USA). 2x150bp Illumina reads were mapped to a proprietary microbial genome database for taxonomic profiling (Thoendel M et al. Journal of Clinical Microbiology 2020;58(3):e00981-19) and to UniRef90 and MetaCyc for pathway profiling (Franzosa EA et al. Species-level functional profiling of metagenomes and metatranscriptomes. Nature Methods 2018;15(11):962-8). Treatment induced microbial composition modulation was assessed with both unsupervised analysis (multi-dimensional scaling using UniFrac distances) and supervised analysis (sparse partial least-squares discriminant analysis, sPLS-DA), using the R packages phyloseq (McMurdie PJ, Holmes S. PLoS ONE 2013;8(4):e61217) and mixOmics (Rohart F. PLOS Computational Biology 2017;13(11):e1005752), respectively. Classifier performance for the latter was appreciated through the balanced error rate (BER).

$$BER = 0.5 \times \left( \frac{FP}{TN + FP} + \frac{FN}{TP + FN} \right)$$

*Liver transcriptomics*

RNA extraction, RNA library preparations, and sequencing reactions were conducted at GENEWIZ, LLC. (South Plainfield, NJ, USA) as follows: total RNA was extracted from fresh frozen liver tissue samples using the Qiagen RNeasy Plus Universal mini kit following manufacturer's instructions (Qiagen, Hilden, Germany). RNA samples were quantified using Qubit 2.0 Fluorometer (Life Technologies, Carlsbad, CA, USA), and RNA integrity was measured using the RNA Screen Tape on Agilent 2200 TapeStation (Agilent Technologies, Palo Alto, CA, USA). RNA sequencing libraries were prepared using TruSeq Stranded mRNA library prep kit following manufacturer's protocol (Illumina, Cat# RS-122-2101). First, mRNA's were enriched with Oligo d(T) beads. Enriched mRNA's were fragmented for 8 minutes at 94 °C. Subsequently, first strand and second strand cDNA was synthesized. The second strand of cDNA was marked by incorporating dUTP's during synthesis. cDNA fragments were adenylated at 3'ends and indexed adapters were ligated to cDNA fragments. Limited cycle PCR was used for library enrichment. The incorporated dUTP's in second strand cDNA quenched the amplification thus preserving strand specificity. Sequencing libraries were validated using DNA Analysis Screen Tape on the Agilent 2200 TapeStation (Agilent Technologies, Palo Alto, CA, USA), and quantified by using Qubit 2.0 Fluorometer (Invitrogen, Carlsbad, CA) as well as by quantitative PCR (KAPA Biosystems, Wilmington, MA, USA). The pooled libraries were clustered on 7 lanes of a flowcell. After clustering, the flowcell was loaded on the Illumina HiSeq instrument (4000 or equivalent) according to manufacturer's instructions and sequenced using a 2x150bp Paired End (PE) configuration. Image analysis and base calling were conducted by the HiSeq Control Software (HCS). Raw sequence data (.bcl files) generated from Illumina HiSeq was converted into fastq files and de-multiplexed using Illumina's bcl2fastq 2.17 software. One mismatch was allowed for index sequence identification.

Strand-specific gene expression profiling was achieved through mapping of Illumina 2x150bp reads to the Ensembl *Rattus norvegicus* Rnor_6.0 transcriptome and processing the alignments with featureCounts from the subread package (Liao Y et al. Bioinformatics 2014;30(7):923-30). Rat Ensembl gene IDs were mapped to human Ensembl gene equivalents and HUGO Gene Nomenclature Committee (HGNC) symbols through Ensembl Biomart. Resulting uniquely mapping fragment counts were taken into differential gene expression analysis with the R package DESeq2 (Anders S, Huber W. Genome Biology 2010;11(10):R106) to obtain differentiall expressed genes with Benjamini & Hochberg false discovery rate (FDR) adjusted p-values. Outlier analysis was performed with orthogonal partial least squares analysis using the R package ropls (Thévenot EA. Journal of Proteome Research 2015;14(8):3322-35). Simultaneously, sPLS-DA was performed to estimate variable importance (VIP). Liver genes discriminant for treatment vs. vehicle contrasts were retained as having a VIP > 1 and adjusted p-value < 0.05. Functional over-representation analysis (ORA) in these genes was performed with the R package clusterProfiler (Wu T et al. The Innovation 2021;2(3):100141), using the WikiPathways functional annotation database (Martens M et al. Nucleic Acids Research 2021;49(D1):D613-21.). The R package RcisTarget (Aibar S et al. SCENIC: Single-cell regulatory network inference and clustering. Nature Methods 2017;14(11):1083-6) was used to predict transcription motifs enriched in under- or over-expressed genes. High confidence transcription factors associated with these motifs were retained.

*Biological parameter analysis*

Continuous variables were documented as mean $\pm$ standard error of the mean (SEM). Statistical analyses were performed using GraphPad Prism software (GraphPad Software, San Diego, CA). Biological parameters of treatment groups were compared by Student's t-test or One Way ANOVA with Dunnett's multiple comparisons' test. A P-value < 0.05 was considered statistically significant.

Results

*Consortium treatment significantly improved body weight*

During the 2-week treatment period, animals in the HFGFD-V group increased their body weight to a great extent (22.08 $\pm$ 3.18g), as seen in Figure 2A. However, HFGFD-C only gained on average 2.91 $\pm$ 3.55 g, whereas HFGFD-Tr lost on average 5 $\pm$ 3.47 g. Compared to the HFGFD-V, both groups receiving microbiota-based treatments gained significantly less weight during the 2-week treatment period (Figure 2A).

*Consortium treatment significantly improved the metabolic profile*

As seen in Figure 2B and C and Table 2, both microbiota-based treatments displayed important reductions in fasting blood insulin levels and HOMA-IR index with respect to the HFGFD-V group.

Table 2: Biochemical characteristics after 2 weeks of intervention.

|  | HFGFD-V N=13 | HFGFD-C N=11 | HFGFD-Tr N=11 |
|---|---|---|---|
| **Body weight pre-trt. (g)** | 531.7 $\pm$ 10.3 | 543.6 $\pm$ 10.29 | 515.1 $\pm$ 13.48 |
| **Body weight gain with trt. (g)** | 22.08 $\pm$ 3.18 | **2.91 $\pm$ 3.55\*** | **-5 $\pm$ 3.47\*** |
| **Body weight post-trt. (g)** | 552.33 $\pm$ 11.77 | 546.6 $\pm$ 11.53 | **507 $\pm$ 12.39\*** |
| ***BIOCHEMISTRY*** |  |  |  |
| **Glucose (mg/dL)** | 182.45 $\pm$ 14.94 | 166.9 $\pm$ 14.89 | 171.36 $\pm$ 10.47 |
| **Insulin (ng/mL)** | 15.36 $\pm$ 3.6 | 9.24 $\pm$ 1.17 | 11.21 $\pm$ 1.13 |
| **HOMA-IR** | 7.89 $\pm$ 2.3 | 3.7 $\pm$ 0.54 | 4.68 $\pm$ 0.45 |
| **Albumin (g/dL)** | 2.9 $\pm$ 0.04 | 3.03 $\pm$ 0.05 | **3.13 $\pm$ 0.06\*** |
| **Bilirubin (mg/dL)** | 0.09 $\pm$ 0.01 | 0.09 $\pm$ 0.02 | 0.11 $\pm$ 0.01 |
| **AST (IU/L)** | 205.56 $\pm$ 34.05 | 234.29 $\pm$ 45.72 | 172 $\pm$ 63.52 |
| **ALT (IU/L)** | 66.15 $\pm$ 4.36 | 67.1 $\pm$ 9.29 | **47.5 $\pm$ 31.31\*** |
| **TG (mg/dL)** | 27.92 $\pm$ 1.35 | **33 $\pm$ 1.91\*** | **33.54 $\pm$ 2.96\*** |
| **Cholesterol (mg/dL)** | 75.54 $\pm$ 4.75 | 86.78 $\pm$ 3.89 | 78.91 $\pm$ 2.99 |
| **Cholesterol HDL (mg/dL)** | 41.93 $\pm$ 2.43 | 46.78 $\pm$ 1.97 | 45.54 $\pm$ 2.48 |
| **Cholesterol LDL (mg/dL)** | 25.27 $\pm$ 2.52 | 34.75 $\pm$ 3.4 | 27 $\pm$ 1.96 |

*P $\leq$ 0.05 *versus* HFGFD-V. Abbreviations: ALT, alanine aminotransferase; AST, aspartate aminotransferase; HDL, high-density lipoprotein; HFGFD, high-fat, high-glucose/fructose diet; HOMA-IR, Homeostasis model of insulin resistance index; LDL, low-density lipoprotein; TG, triglycerides.

*Reduced expression of pro-fibrotic hepatic marker genes*

At the point when animals were sacrificed (10 weeks on HFGFD) pro-fibrotic pathways are already activated, and gene expression of pro-fibrotic hepatic markers $\alpha$-*SMA* and *COL1A1* were significantly reduced in the consortium-treated group when compared with the vehicle group (Figure 3). These results are further corroborated by the genome-wide RNA-Seq analysis results discussed below.

*Consortium treatment significantly reduced portal pressure and ameliorated liver hemodynamics*

Consistent with previous findings (9), and despite the absence of histological fibrosis, animals in a 10-week HFGFD intervention showed increased portal pressure (PP) with values of 10.32 mmHg on average (Table 3 and Figure 4A). Importantly, both the bacterial consortium treatment and IM transplantation significantly reduced PP when compared to the vehicle-treated group, even while maintaining the HFGFD during the 2-week treatment period (Table 3 and Figure 4A). This decrease in PP observed in the HFGFD-C group was secondary to a significant decrease (80% reduction) in the intrahepatic vascular resistance (IHVR) as compared to HFGFD-V, in a similar degree as measured in the IM-Tr group (104% reduction) (Figure 4B). Regarding systemic hemodynamics, no relevant changes were observed between groups (Table 3).

Table 3: Hemodynamic measurements after 2 weeks of intervention.

| | HFGFD-V N=13 | HFGFD-C N=11 | HFGFD-TR N=11 |
|---|---|---|---|
| MAP (mmHg) | 113.05 ± 4.53 | 119.95 ± 4.21 | 117.34 ± 5.14 |
| PP (mmHg) | 10.32 ± 0.22 | **9.58 ± 0.19*** | **9.21 ± 0.12*** |
| SMABF (mL/[min * 100 g]) | 2.73 ± 0.24 | 2.71 ± 0.21 | 2.82 ± 0.17 |
| SMAR (mmHg/mL * min * 100 g) | 40.78 ± 3.25 | 40.79 ± 4.36 | 39.35 ± 2.36 |
| IHVR (mmHg/mL*min*100 g) | 7.58 ± 1.27 | **4.20 ± 0.35*** | **3.71 ± 0.21*** |

*$P \leq 0.05$ versus HFGFD-V. Abbreviations: HFGFD, high-fat, high-glucose/fructose diet; IHVR, intrahepatic vascular resistance; MAP, mean arterial pressure; PP, portal pressure; SMABF, superior mesenteric artery blood flow; SMAR, superior mesenteric artery resistance.

*Features of endothelial dysfunction (ED) were improved in animals after receiving consortium treatment*

ED molecular hallmarks were assessed to further characterize the changes observed in IHVR and PP induced by the microbiota-based treatments with respect to the vehicle-treated group. For this purpose, we analyzed the phosphorylated protein kinase B (P-Akt) and endothelial nitric oxide synthase (P-eNOS). In addition, to evaluate the possible transcriptional regulation mechanisms of e-NOS, KLF2 was evaluated as it is considered essential to maintain the functional endothelial phenotype. As seen in Figure 4C-E, microbiota-based treatments induced a significant increase in P-Akt. P-eNOS showed a tendency to increase in both HFGFD-C and HFGFD-Tr when compared to HFGFD-V. Interestingly, HFGFD-C was unique in showing a marked significant increase in KLF2.

*Microbiota based treatments improve caecal species diversity and drive important cecal microbiome composition shifts*

As shown by the inverse Simpson index from the phyloseq package (Figure 5), microbiota based treatments improve caecal species diversity. One outlier in the microbial consortium treatment group was identified, rat R72 from the second batch.

Beta diversity as appreciated through multi-dimensional scaling applied without the CD group shows good separation of the HGHFD-V, HGFD-C and HGFD-Tr groups (Figure 6).

Classifier performance as appreciated through the BER for the HFGFD-C contrast (0.03) compares favorably to the HFGFD-Tr contrast (0.34), Figure 7A. Hence it can be argued HFGFD-C induces a more defined change than HFGFD-Tr, but this can be dominated by or even limited to the addition of product strains. Yet the corresponding feature selection (VIP>1, n=123) shows that the bacterial consortium treatment induced suppression of 72 (58.5% of observable change) vehicle associated taxa and promoted the emergence of 48 (41.5%) non-product treatment associated taxa, whereas the microbiota transplantation according to feature selection (n=178) induced suppression of 135 (75.8%) taxa and their replacement with 43 (24.2%) taxa. Hence both treatments operated an important replacement of the HGHFD-V associated microbial community, but the bacterial consortium achieved a more defined and richer replacement than the transplantation, whereas the transplantation was more suppressive in nature, possibly due to the intestinal emptying procedure done in this group before transplant to improve engraftment. Figure 7B provides the top 10 discriminative taxa according to classifier estimated variable importance (VIP) and their associations, whereas Figure 7C combines the three V-C, V-Tr and C-Tr contrasts into a single Euler plot proportional to the numbers of features selected for each contrast.

*Bacterial consortium treatment induce differential liver gene expression and down-regulate fibrosis associated genes*

The expression of 32,552 liver genes was documented through RNA-Seq analysis. Of these, 732 (2.2%) were discriminant (VIP>1, FDR p-value < 0.05) for either the HFHGD-C (n=252) or HFHGD-Tr (n=632) contrasts, or both. For the HGHFD-C, there was a trend towards the up-regulation of treatment associated genes (n=157, 62.3%), whereas for HGHFD-Tr there was a balance between up-regulation of treatment associated genes (n=315, 49.8%) and down-regulation of vehicle associated genes (n=317, 50.4%).

We performed overrepresentation analysis (ORA) on genes up-regulated in either or both HFHGD-C or HFHGD-Tr, which did not detect any significant pathway over-representation against the WikiPathways annotation database. The same analysis performed on down-regulated genes detected several pathways associated with fibrosis.

Example 2: comparison of a 6-strain consortium and a 9-strain consortium in the rat NASH model of portal hypertension (PHT)

Example 2 has been conducted using the same materials and methods as described for Example 1. The 9-strain consortium showed consistent results across the multiple endpoints. The 6-strain consortium also induced significant protective effects, particularly on fasting insulin levels, HOMA-IR index, and liver hemodynamics (PP, IHVR) (Figure 8).

In the 6-strain consortium-treated group phosphorylated (p)-AKT and p-eNOS did not really change in response to treatment (Figure 9A and B), which suggests that other mechanisms may be at play to improve insulin resistance and thereby portal hypertension; in addition, the increase in the expression of KLF2 seems to be specific for the 9-strain consortium (Figure 9C).

Therefore, despite their similar composition, the effects of these two consortia were not totally comparable. Particularly, the addition of (further) strains capable of producing propionate appears to be advantageous and may activate additional beneficial mechanisms.

Example 3: STAM™ mouse model of NASH

Materials and methods

*Experimental design*

The STAM™ study was conducted by SMC Laboratories in Japan. Briefly, NASH was induced in C57BL/6J male mice by a single subcutaneous injection of 200 $\mu$g streptozotocin (STZ, Sigma-Aldrich, USA) solution 2 days after birth, and by introducing a high-fat diet (HFD, 57 kcal% fat, Cat# HFD32, CLEA Japan, Inc., Japan) at 4 weeks of age.

The STAM mouse model has the substantial benefit of its HCC development from NASH being identical to the known progression in human patients, but with the whole process being completed within a relatively short period of time (Fujii, M. et al. Med. Mol. Morphol. 2013;46:141-152). In this model, male C57BL/6 mice aged two days are given a single dose of streptozotocin (STZ) to reduce insulin secretory capacity. At 4 weeks of age, they start a high-fat diet (HFD) feeding. The model can be summarized as follows (Table 4):

Table 4: Disease progression in the STAM™ mouse model of NASH.

| 0 wks | birth |  |
|---|---|---|
| 2 days | First hit: induction of beta-cell injury by STZ |  |
|  |  | • beta cell injury early after birth drives regenerative responses with islet inflammation |

(continued)

| | |
|---|---|
| | • This drives accumulation of macrophages in the islet and adipose tissue<br>• And induction of a mild diabetic condition<br>• There is also upregulation of scavenger receptors and TNFalpha in the liver ("priming") |
| 4 wks | 2nd hit (continuous HFD)<br><br>• HFD augments fat deposition in the primed liver with increased lipogenesis<br>• fatty acid oxidation induces ROS generation, lipid peroxidation and mitochondria dysfunction<br>• Recruitment and activation of inflammatory cells (macrophages followed by fibroblasts)<br>• Proliferation of hepatocytes and tumor formation |
| 5-6 wks | Steatosis |
| 6 wks | increased NAFLD Activity Score (NAS) at histology and elevated ALT levels |
| 7-8 wks | Steatohepatitis |
| 9-12 wks | Fibrosis (to chronic phase) |
| 16 wks | HCC |

Two days after birth, C57BL/6J mice are injected with STZ, a beta-cell toxin. This primes the animals, so that when the second hit is initiated at 4 weeks of age, i.e., initiation of a continuous high-fat diet (HFD), disease progresses rapidly. By 5 weeks of age there is hepatic lipid accumulation (steatosis), and by 6 weeks animals have an increased NAFLD Activity Score (NAS) at histology and elevated ALT levels. By 7 to 8 weeks of age hepatic inflammation is evident, and at 9 to 12 weeks, fibrosis is apparent. HCC can be observed approx. at 16 weeks of age. This model is concordant with late T2D that progresses into fatty liver, NASH, fibrosis, and consequently liver cancer (HCC). Compared to other NASH-HCC models, disease progresses in a relatively short period of time, and liver cancer is developed in all animals by 20 weeks of age. An advantage of this model is the rapid disease onset and progression and the complete penetrance of the phenotype. According to the targeted phase of disease progression, animals are euthanized at earlier or later stages of the disease. The model displays notable signs of fibrosis at histopathology.

Animals were randomized into three treatment groups based on their body weight the day before feeding HFD. Randomization was performed by body weight-stratified random sampling using Excel software. At 5 weeks of age (after one week on HFD) animals received either sham gavage (STAM+VEH, n = 16), the 9-strain bacterial consortium (STAM+CON, n = 18), or Telmisartan (STAM+TLM, n = 8). A control group fed regular diet and receiving sham gavage was also included in this study (CD+VEH, n = 8) (Figure 10). Animals were maintained in a SPF facility under controlled conditions of temperature ($23 \pm 3°C$), humidity ($50 \pm 20\%$), lighting (12-hour artificial light and dark cycles; light from 8:00 to 20:00) and air exchange. This animal experiment was conducted according to the following guidelines: Act on Welfare and Management of Animals (Ministry of the Environment, Act No. 105 of October 1, 1973, Japan; Standards Relating to the Care and Management of Laboratory Animals and Relief of Pain (Notice No.88 of the Ministry of the Environment, April 28, 2006, Japan) and Guidelines for Proper Conduct of Animal Experiments (Science Council of Japan, June 1, 2006).

*Treatment administration*

Vehicle (sterile PBS) and bacterial consortium were administered orally in a volume of 200 μl/mouse; the consortium was administered at the dose of $10^9$ CFU/day for a total of 4 weeks. Product suspensions were prepared as described above. Telmisartan was used as benchmark and administered orally at the daily dose of 10 mg/kg in a volume of 10 ml/kg for 4 weeks. For that, one tablet of Telmisartan was transferred into a mortar and triturated with pestle by adding pure water until obtaining a homogenous suspension of 1 mg/ml. All treatments were prepared freshly prior to administration. Body weight was recorded daily during the experimental period.

*Sacrifice and sample collection*

Animals belonging to the control (CD+VEH) and STAM groups (STAM+VEH, STAM+CON, and STAM+TLM) were sacrificed after 4 weeks of treatment, i.e., at 9 weeks of age, which corresponds to a steatohepatitis/early fibrosis stage. Besides, two groups of STAM animals belonging to the vehicle and consortium groups were followed for additional three weeks and were sacrificed at 12 weeks of age, which corresponds to a stage of late fibrosis. This was performed to investigate the potential of the bacterial consortium to delay disease progression upon cessation of treatment (Figure 10). Animals were sacrificed by exsanguination through direct cardiac puncture under isoflurane anesthesia (Pfizer Inc.). Non-fasting blood was drawn from the facial vein in polypropylene tubes with anticoagulant (Novo-heparin, Mochida Pharmaceutical, Japan). Fifty (50) $\mu$L of collected blood was used for glycated hemoglobin (HbA1c) analysis. Non-fasting blood was also collected for serum preparation in separate tubes without anticoagulant. These were incubated at room temperature for 30 minutes, and then centrifuged at 3,500 xg for 5 minutes at 4 °C. Remaining serum was collected and stored at -80 °C until further use for the quantification of serum CK-18 levels and biochemistry. Whole liver was collected and washed with cold saline. Liver specimens were stored at -80 °C embedded in Optimal Cutting Temperature (O.C.T., Sakura Finetek Japan, Japan) compound for immunohistochemistry, or fixed in Bouin's solution (Sigma-Aldrich Japan, Japan) for 24 hours. After fixation, these specimens proceeded to paraffin embedding for H&E and Sirius-red staining. The O.C.T.-embedded specimens were used for F4/80 immunohistochemistry (see below).

*Blood biochemistry*

HbA1c in whole blood was quantified by DCN2000+ (Siemens Healthcare Diagnostics, USA). Serum CK-18 levels were measured by using the Mouse Cytokeratin 18-M30 ELISA kit (Cusabio Biotech Co., Ltd, China). Serum alanine aminotransferase (ALT) and triglyceride levels were measured by FUJI DRI-CHEM 7000 (Fujifilm Corporation). Serum total cholesterol, high-density lipoprotein (HDL)-cholesterol and low-density lipoprotein (LDL)-cholesterol levels were quantified by HPLC at Skylight Biotech Inc. (Japan).

*Histological analyses*

Sections were cut from paraffin blocks of liver tissue using the rotary microtome (Leica Microsystems). After sectioning, each slide was coded a number for blind evaluation. Each number was generated using the RAND function of Excel software, sorted in ascending order and assigned the slides. The tissue slides were used for the following stains and evaluated by an experimenter: for H&E staining, sections were cut from paraffin blocks of liver tissue prefixed in Bouin's solution and stained with Lillie-Mayer's Hematoxylin (Muto Pure Chemicals Co., Ltd., Japan) and eosin solution (FUJIFILM Wako Pure Chemical Corporation, Japan). NAFLD Activity score (NAS) was calculated according to the criteria of Kleiner.(22) For scoring of NAS, bright field image of H&E-stained sections was captured using a digital camera (DFC295; Leica, Germany) at 50- and 200-fold magnifications. Steatosis score in 1 section/mouse (representative 1 field at 50-fold magnification), inflammation score in 1 section/mouse (representative 1 field around the central vein at 200-fold magnification) and ballooning score in 1 section/mouse (representative 1 field around the central vein at 200-fold magnification) were estimated. To visualize collagen deposition, Bouin's fixed liver sections were stained with picro-Sirius red solution (Waldeck, Germany). Briefly, sections were deparaffinized and hydrophilized with xylene, 100-70% alcohol series, and RO water, and then treated with 0.03% picro-Sirius red solution (Cat No.: 1A-280) for 60 minutes. After washing through 0.5% acetic acid solution and RO water, stained sections were dehydrated and cleared with 70-100% alcohol series and xylene, then sealed with Entellan™ (Merck, Germany) and used for observation. For quantitative analysis of fibrosis area, bright field images of Sirius red-stained section were captured around the central vein using a digital camera (DFC295; Leica, Germany) at 200-fold magnification, and the positive areas in 5 fields/section were measured using ImageJ software (National Institute of Health, USA).

For immunohistochemistry, sections were cut from frozen liver tissues embedded in Tissue-Tek O.C.T. compound and fixed in acetone. Endogenous peroxidase activity was blocked using 0.03% $H_2O_2$ for 5 minutes, followed by incubation with Block Ace (Dainippon Sumitomo Pharma Co. Ltd., Japan) for 10 minutes. Sections were incubated with anti-F4/80 antibody overnight (Monoclonal Antibody, T-2006, BMA Biomedicals) at 4°C. After incubation with secondary antibody (VECTASTAIN ABC KIT, PK-4004, Vector Laboratories), enzyme-substrate reactions were performed using 3,3'-diaminobenzidine/$H_2O_2$ solution (Nichirei Bioscience Inc., Japan). Bright field images of F4/80-immunostained sections were captured around the central vein using a digital camera (DFC295; Leica, Germany) at 200-fold magnification, and the positive areas in 5 fields/section were measured using ImageJ software (National Institute of Health, USA).

Results

Because the liver transcriptomics performed in the NASH rats suggested that the tested bacterial consortium could be favorably modulating pro-fibrogenic pathways, we prompted to test the same consortium on a rodent model that displays notable signs of fibrosis at histopathology. For that, the STAM™ mouse model was used. In this model, male mice were injected subcutaneously shortly after birth with STZ, a β-cell toxin that induces mild islet inflammation and destruction. In addition, STZ-primed mice initiated a continuous HFD at 4 weeks of age. This induces sequential histological changes from fatty liver, to NASH, fibrosis and finally, tumor protrusion. Disease progresses rapidly, with nearly 100% penetrance. This allows studying the protective effects of drugs at well-defined time points, depending on the targeted disease stage. For a fibrosis-targeting study, animals can be sacrificed between 8 and 12 weeks of age. Thus, the effects of the 9-strain consortium were evaluated at the end of the 4-week treatment period, i.e., at the stage of steatohepatitis/early fibrosis (9 weeks of age), and also at 12 weeks of age, which is concomitant with late fibrosis. Results were compared to the benchmark Telmisartan, an angiotensin II receptor blocker that decreases hepatic fat accumulation, and inhibits hepatic stellate cell activation and thus suppresses hepatic fibrogenesis. The effects were evaluated at histopathological level and blood was collected for biochemical analysis.

A trend to improve body weight gain by the end of the study, i.e., between 9 and 12 weeks of age, was observed for the consortium-treated group (Figure 11). However, the STAM™ model is not the most appropriate to follow obesity as STAM animals are lean, which is contrast with other models of diet-induced NASH. Similar to the NASH rats, no significant alterations were observed in blood parameters, namely ALT and cholesterol levels (Table 5).

A reduction in whole blood levels of HbA1cv (glycated hemoglobin) was also observed by the end of the study, indicating a beneficial effect on diabetes (Figure 12).

Table 5: Biochemical characteristics after 4 weeks of intervention in the STAM mouse study.

| | 9 WEEKS | | | | 12 WEEKS | |
|---|---|---|---|---|---|---|
| | CD+VEH | STAM+VEH | STAM+CON | STAM+TLM | STAM+VEH | STAM+CONN |
| | n=8 | n=8 | n=10 | N=8 | N=8 | N=8 |
| ALT (IU/L) | 24.00*** ± 1.27 | 39.38 ± 2.41 | 43.5 ± 3.28 | 35.00 ± 2.39 | 65.13 ± 14.04 | 60.63 ± 12.81 |
| TG (MG/DL) | 79.63* ± 10.46 | 500.9 ± 152.5 | 679.9 ± 91.35 | 465.6 ± 107.6 | 926.4 ± 251.6 | 1017 ± 248.8 |
| TOTAL CHOLESTEROL (MG/DL) | 76.92*** * ± 3.51 | 125.7 ± 8.11 | 124.8 ± 4.32 | 132.7 ± 2.71 | 187.3 ± 41.99 | 186.9 ± 38.81 |
| CHOLESTEROL HDL (MG/DL) | 57.84** ± 2.75 | 83.69 ± 7.13 | 75.55 ± 3.31 | 97.21 ± 4.73 | 72.24 ± 7.46 | 70.21 ± 6.84 |
| CHOLESTEROL LDL (MG/DL) | 13.1 ± 0.66 | 14.68 ± 1.20 | 13.64 ± 0.74 | 18.78* ± 1.10 | 24.35 ± 6.12 | 23.76 ± 5.16 |

*,**,***, and **** P ≤0.05, ≤0.01, ≤0.001, and ≤0.0001, respectively, *versus* STAM+VEH. Abbreviations: ALT, alanine aminotransferase; HDL, high-density lipoprotein; LDL, low-density lipoprotein; TG, triglycerides.

*The consortium of 9 gut commensals improved NAS at histopathology at 9 weeks of age*

Liver sections were collected for evaluation of the hallmarks of NAFLD, i.e., hepatic inflammation, steatosis, and ballooning. Representative images of H&E-stained liver sections are shown in Figure 13; calculated scores are depicted in Figure 14. As expected, liver sections from the vehicle-treated STAM group exhibited micro- and macrovesicular fat deposition, hepatocellular ballooning and inflammatory cell infiltration. The bacterial consortium-treated STAM mice displayed significantly reduced NAS scores when compared to the disease group at 9 weeks of age (Figure 14D). This was a result from reduced steatosis and ballooning scores (Figure 14B and C). At 12 weeks of age this was no longer observed.

*The consortium of 9 gut commensals improved fibrosis, and showed reduced hepatic expression of F4/80 and serum CK-18 levels*

To evaluate collagen deposition, liver sections were stained with picro-Sirius red. Representative images are shown in Figure 15A. Calculated areas are depicted in Figure 16A. As expected, liver sections from the vehicle-treated STAM group displayed increased collagen deposition in the pericentral region of the liver lobule when compared to the control diet group. The bacterial consortium-treated group showed a significant decrease in the fibrosis (Sirius red-positive) area when compared to the disease group at 12 weeks of age but not at 9 weeks of age. To further evaluate inflammation, the macrophage F4/80 immunostaining was used in liver sections. Despite the fact that no significantly reduced general inflammation was observed in H&E-stained sections (Figure 14A and Figure 13), the F4/80 stained sections showed a significant reduction in macrophage inflammatory infiltration in the consortium-treated group at 9 weeks of age, and a trend to decrease at 12 weeks of age (Figure 16B and Figure 15B). Finally, CK-18 serum levels were also significantly reduced in the consortium-treated group at 9 weeks of age, and a trend to decrease was observed by 12 weeks of age (Figure 16C), a result that nicely correlates with the histological activity.

These results confirm that indeed the 9-strain consortium can prevent NASH disease progression to fibrosis.

Example 4

SCFA production of *Phocaeicola vulgatus* (strain LMG17767), *Veillonella parvula* (strain DSM 2007), and *Blautia obeum* (strain DSM 25238) was measured and compared with alternative strains of the same or related species:

| | |
|---|---|
| Alternatives for *Phocaeicola vulgatus* (strain LMG17767):<br>• *P. vulgatus* PD301<br>• *P. vulgatus* PD380<br>• *P. vulgatus* PD489 | Growth conditions LMG17767:<br>• Media: HV4S<br>• 48h of incubation<br>• Anaerobic conditions at 37°C<br>Growth conditions PD301:<br>• Media: HV5<br>• 24h of incubation<br>• Anaerobic conditions at 37°C<br>Growth conditions PD380 and PD489:<br>• Media: GAM<br>• 24h of incubation<br>• Anaerobic conditions at 37°C |
| Alternatives for *Veillonella parvula* (strain DSM 2007):<br>• *V. atypica* PD219<br>• *V. atypica* PD220<br>• *V. atypica* PD221<br>• *V. atypica* PD222<br>• *V. atypica* PD260 | Growth conditions:<br>• Media: HV4S<br>• 48h of incubation<br>• Anaerobic conditions at 37°C |
| Alternatives for *Blautia obeum* (strain DSM 25238):<br>• *B. wexlerae* PD393<br>• *B. luti* PD338 | Growth conditions DSM 25238:<br>• Media: HV4S<br>• 48h of incubation<br>• Anaerobic conditions at 37°C<br>Growth conditions PD393 and PD338:<br>• Media: HV5<br>• 24h of incubation<br>• Anaerobic conditions at 37°C |

All tested strains are commensal gut bacteria isolated from faecal material of healthy human donors. SCFA levels were quantified by Gas Chromatography (GC).

All tested strains show a highly similar SCFA profile (Fig. 17), indicating that the consortium results described above can also be obtained by replacing *Phocaeicola vulgatus* (strain LMG17767), *Veillonella parvula* (strain DSM 2007), and/or *Blautia obeum* (strain DSM 25238) with alternative strains of the same or related species.

**References**

1. Younossi ZM, Golabi P, de Avila L, Paik JM, Srishord M, Fukui N, et al. The global epidemiology of NAFLD and NASH in patients with type 2 diabetes: A systematic review and meta-analysis. J. Hepatol. 2019;71:793-801.
2. Qian Y, Fan JG. Obesity, fatty liver and liver cancer. Springer; 2018.
3. Li J, Zou B, Yeo YH, Feng Y, Xie X, Lee DH, et al. Prevalence, incidence, and outcome of non-alcoholic fatty liver disease in Asia, 1999-2019: a systematic review and meta-analysis. Lancet Gastroenterol. Hepatol. 2019;4:389-398.

4. Younossi ZM, Tampi R, Priyadarshini M, Nader F, Younossi IM, Racila A. Burden of Illness and Economic Model for Patients With Nonalcoholic Steatohepatitis in the United States. Hepatology. 2019;69:564-572.

5. Chalasani N, Younossi Z, Lavine JE, Charlton M, Cusi K, Rinella M, et al. The diagnosis and management of nonalcoholic fatty liver disease: Practice guidance from the American Association for the Study of Liver Diseases. Hepatology. 2018;67:328-357.

6. Lago RM, Singh PP, Nesto RW. Congestive heart failure and cardiovascular death in patients with prediabetes and type 2 diabetes given thiazolidinediones: a meta-analysis of randomised clinical trials. Lancet (London, England). 2007;370:1129-1136.

7. Lincoff AM, Wolski K, Nicholls SJ, Nissen SE. Pioglitazone and risk of cardiovascular events in patients with type 2 diabetes mellitus: a meta-analysis of randomized trials. JAMA. 2007;298:1180-1188.

8. Nissen SE, Wolski K. Effect of rosiglitazone on the risk of myocardial infarction and death from cardiovascular causes. N. Engl. J. Med. 2007;356:2457-2471.

9. Garcia-Lezana T, Raurell I, Bravo M, Torres-Arauz M, Salcedo MT, Santiago A, et al. Restoration of a healthy intestinal microbiota normalizes portal hypertension in a rat model of nonalcoholic steatohepatitis. Hepatology. 2018;67:1485-1498.

Sequences

| SEQ ID NO: | Description of the sequence |
|---|---|
| 1 | *Roseburia inulinivorans* DSMZ 16841 16S rRNA gene (Genbank: AJ270473.3) |
| 2 | *Akkermansia muciniphila* DSMZ 22959 16S rRNA gene (Genbank: AY271254.1) |
| 3 | *Anaerostipes caccae* DSMZ 14662 16S rRNA gene (Genbank: AJ270487.2) |
| 4 | *Faecalibacterium prausnitzii* DSMZ 17677 16S rRNA gene (Genbank: AJ270469.2) |
| 5 | *Lactiplantibacillus plantarum* ZJ316 16S rRNA gene (Genbank: JN126052.1) |
| 6 | *Butyricicoccus pullicaecorum* LMG 24109 16S rRNA gene (Genbank: HH793440.1) |
| 7 | *Phocaeicola vulgatus* LMG17767 16S rRNA gene (NCBI NR_074515.1) |
| 8 | *Veillonella parvula* DSM 2007 16S rRNA gene (ENA AY995769.1) |
| 9 | *Blautia obeum* DSM 25238 16S rRNA gene (NCBI X85101.1) |

**Claims**

1. A composition comprising:

   at least one bacterial strain of *Faecalibacterium prausnitzii;*
   at least one bacterial strain of *Butyricicocccus pullicaecorum;*
   at least one bacterial strain of *Roseburia inulinivorans;*
   at least one bacterial strain of *Akkermansia muciniphila;*
   at least one bacterial strain of *Lactiplantibacillus plantarum;* and
   at least one bacterial strain of *Anaerostipes caccae,*
   for use in a method of treating, delaying, ameliorating, preventing or curing non-alcoholic fatty liver disease (NAFLD), liver fibrosis, liver cirrhosis and/or hepatocellular carcinoma (HCC).

2. A composition for use according to claim 1, wherein the composition further comprises at least one additional bacterial strain capable of producing propionate or a propionate precursor.

3. A composition for use according to claim 1, wherein the composition further comprises at least two additional bacterial strains capable of producing propionate or a propionate precursor.

4. A composition for use according to claim 1, wherein the composition further comprises three additional bacterial strains capable of producing propionate or a propionate precursor.

5. A composition for use according to any one of claims 2 to 4, wherein the one or more additional bacterial strains capable of producing propionate or a propionate precursor belong to the genus *Phocaeicola, Veillonella* or *Blautia,* preferably to the species *Phocaeicola vulgatus, Veillonella parvula, Veillonella atypica, Blautia obeum, Blautia wexlerae,* or *Blautia luti.*

6. A composition for use according to any one of claims 2 to 5, wherein the composition further comprises:

   - *Phocaeicola vulgatus;*
   - *Veillonella parvula* or *Veillonella atypica;* and
   - *Blautia obeum, Blautia wexlerae,* or *Blautia luti.*

7. A composition for use according to any one of claims 1 to 6, wherein the method results in the reduction of weight gain, insulin resistance, liver inflammation, steatosis, hepatocellular ballooning, intrahepatic vascular resistance (IHVR), portal hypertension, endothelial dysfunction and/or fibrosis.

8. A composition for use according to any one of claims 1 to 7, wherein the composition is for use in a method of delaying or preventing disease progression of NAFL, preferably delaying progression of NAFL to NASH, NASH to liver fibrosis, liver fibrosis to liver cirrhosis and/or liver cirrhosis to HCC.

9. A composition for use according to any one of claims 1 to 8, wherein the composition is administered orally.

10. A composition for use according to claim 9, wherein the composition is formulated as a capsule, microcapsule, tablet, granule, powder, troche, pill, food supplement, suspension or syrup.

11. A composition comprising bacterial strains belonging to the species *Faecalibacterium prausnitzii, Butyricicocccus pullicaecorum, Roseburia inulinivorans, Akkermansia muciniphila, Lactiplantibacillus plantarum, Anaerostipes caccae, Phocaeicola vulgatus, Veillonella parvula,* and *Blautia obeum.*

**Patentansprüche**

1. Zusammensetzung, umfassend:

   mindestens einen Bakterienstamm von *Faecalibacterium prausnitzii;*
   mindestens einen Bakterienstamm von *Butyricicoccus pullicaecorum,*
   mindestens einen Bakterienstamm von *Roseburia inulinivorans;*
   mindestens einen Bakterienstamm von *Akkermansia muciniphila;*
   mindestens einen Bakterienstamm von *Lactiplantibacillus plantarum;* und
   mindestens einen Bakterienstamm von *Anaerostipes caccae,*
   zur Anwendung in einem Verfahren zur Behandlung, Verzögerung, Linderung, Vorbeugung oder Heilung von nichtalkoholischer Fettlebererkrankung (NAFLD), Leberfibrose, Leberzirrhose und/oder Leberzellkarzinom (HCC).

2. Zusammensetzung zur Anwendung nach Anspruch 1, wobei die Zusammensetzung weiter mindestens einen zusätzlichen Bakterienstamm umfasst, der Propionat oder einen Propionatvorläufer produzieren kann.

3. Zusammensetzung zur Anwendung nach Anspruch 1, wobei die Zusammensetzung weiter mindestens zwei zusätzliche Bakterienstämme umfasst, die Propionat oder einen Propionatvorläufer produzieren können.

4. Zusammensetzung zur Anwendung nach Anspruch 1, wobei die Zusammensetzung weiter drei zusätzliche Bakterienstämme umfasst, die Propionat oder einen Propionatvorläufer produzieren können.

5. Zusammensetzung zur Anwendung nach einem der Ansprüche 2 bis 4, wobei der eine oder die mehreren zusätzlichen Bakterienstämme, die Propionat oder einen Propionatvorläufer produzieren können, zu der Gattung *Phocaeicola, Veillonella* oder *Blautia* gehören, vorzugsweise zu den Spezies *Phocaeicola vulgatus, Veillonella parvula, Veillonella atypica, Blautia obeum, Blautia wexlerae* oder *Blautia luti.*

6. Zusammensetzung zur Anwendung nach einem der Ansprüche 2 bis 5, wobei die Zusammensetzung weiter Folgendes umfasst:

- *Phocaeicola vulgatus;*
- *Veillonella parvula* oder *Veillonella atypica;* und
- *Blautia obeum, Blautia wexlerae* oder *Blautia luti.*

7. Zusammensetzung zur Anwendung nach einem der Ansprüche 1 bis 6, wobei das Verfahren zu einer Verringerung der Gewichtszunahme, der Insulinresistenz, der Leberentzündung, der Steatose, der hepatocellulären Ballonierung, der intrahepatischen vaskulären Resistenz (IHVR), der portalen Hypertonie, der Endothel-Dysfunktion und/oder der Fibrose führt.

8. Zusammensetzung zur Anwendung nach einem der Ansprüche 1 bis 7, wobei die Zusammensetzung zur Anwendung in einem Verfahren zur Verzögerung oder Verhinderung des Krankheitsfortschreitens von NAFL, vorzugsweise zur Verzögerung des Fortschreitens von NAFL zu NASH, NASH zu Leberfibrose, Leberfibrose zu Leberzirrhose und/oder Leberzirrhose zu HCC, bestimmt ist.

9. Zusammensetzung zur Anwendung nach einem der Ansprüche 1 bis 8, wobei die Zusammensetzung oral verabreicht wird.

10. Zusammensetzung zur Anwendung nach Anspruch 9, wobei die Zusammensetzung als Kapsel, Mikrokapsel, Tablette, Granulat, Pulver, Lutschtablette, Pille, Nahrungsergänzungsmittel, Suspension oder Sirup formuliert ist.

11. Zusammensetzung, umfassend Bakterienstämme, die zu den Spezies *Faecalibacterium prausnitzii, Butyricicoccus pullicaecorum, Roseburia inulinivorans, Akkermansia muciniphila, Lactiplantibacillus plantarum, Anaerostipes caccae, Phocaeicola vulgatus, Veillonella parvula* und *Blautia obeum* gehören.

**Revendications**

1. Composition comprenant:

au moins une souche bactérienne de *Faecalibacterium prausnitzii ;*
au moins une souche bactérienne de *Butyricicocccus pullicaecorum* ;
au moins une souche bactérienne de *Roseburia inulinivorans* ;
au moins une souche bactérienne *d'Akkermansia muciniphila* ;
au moins une souche bactérienne de *Lactiplantibacillus plantarum* ; et
au moins une souche bactérienne *d'Anaerostipes caccae,*
pour l'utilisation dans un procédé de traitement, de retardement, d'amélioration, de prévention ou de guérison de la stéatose hépatique non alcoolique (NAFLD), de la fibrose du foie, de la cirrhose du foie et/ou du carcinome hépatocellulaire (HCC).

2. Composition pour l'utilisation selon la revendication 1, où la composition comprend en outre au moins une souche bactérienne supplémentaire capable de produire du propionate ou un précurseur de propionate.

3. Composition pour l'utilisation selon la revendication 1, où la composition comprend en outre au moins deux souches bactériennes supplémentaires capables de produire du propionate ou un précurseur de propionate.

4. Composition pour l'utilisation selon la revendication 1, où la composition comprend en outre trois souches bactériennes supplémentaires capables de produire du propionate ou un précurseur de propionate.

5. Composition pour l'utilisation selon l'une quelconque des revendications 2 à 4, où l'une ou plusieurs souches bactériennes supplémentaires capables de produire du propionate ou un précurseur de propionate appartiennent au genre *Phocaeicola, Veillonella* ou *Blautia,* de préférence aux espèces *Phocaeicola vulgatus, Veillonella parvula, Veillonella atypica, Blautia obeum, Blautia wexlerae* ou *Blautia luti.*

6. Composition pour l'utilisation selon l'une quelconque des revendications 2 à 5, où la composition comprend en outre :

- *Phocaeicola vulgatus* ;
- *Veillonella parvula* ou *Veillonella atypica* ; et
- *Blautia obeum, Blautia wexlerae* ou *Blautia luti.*

7. Composition pour l'utilisation selon l'une quelconque des revendications 1 à 6, où le procédé entraîne une réduction de la prise de poids, de la résistance à l'insuline, de l'inflammation du foie, de la stéatose, du ballonnement hépatocellulaire, de la résistance vasculaire intrahépatique (IHVR), de l'hypertension portale, du dysfonctionnement endothélial et/ou de la fibrose.

8. Composition pour l'utilisation selon l'une quelconque des revendications 1 à 7, où la composition est pour l'utilisation dans un procédé de retardement ou de prévention de la progression de la maladie de la NAFL, de préférence de retardement de la progression de la NAFL en NASH, de la NASH en fibrose du foie, de la fibrose du foie en cirrhose du foie et/ou de la cirrhose du foie en HCC.

9. Composition pour l'utilisation selon l'une quelconque des revendications 1 à 8, où la composition est administrée par voie orale.

10. Composition pour l'utilisation selon la revendication 9, où la composition est formulée sous forme de capsule, de microcapsule, de comprimé, de granulé, de poudre, de pastille, de pilule, de complément alimentaire, de suspension ou de sirop.

11. Composition comprenant des souches bactériennes appartenant aux espèces *Faecalibacterium prausnitzii, Butyricicocccus pullicaecorum, Roseburia inulinivorans, Akkermansia muciniphila, Lactiplantibacillus plantarum, Anaerostipes caccae, Phocaeicola vulgatus, Veillonella parvula* et *Blautia obeum.*

**FIG 1**

FIG 2

FIG 3

**FIG 4**

D

E

**FIG 4 (continued)**

FIG 5

**Multi-dimensional scaling**

FIG 6

A

B

| taxon | VIP | accociation | contrast |
|---|---|---|---|
| A. caccae DSM 14662 | 3.39 | HFGFD-C | V-C |
| P. vulgatus ATCC 8482 | 3.33 | HFGFD-C | C-Tr |
| P. vulgatus ATCC 8482 | 3.23 | HFGFD-C | V-C |
| B. obeum ATCC 29174 | 3.15 | HFGFD-C | V-C |
| A. caccae DSM 14662 | 2.98 | HFGFD-Tr | V-Tr |
| B. obeum ATCC 29174 | 2.95 | HFGFD-C | C-Tr |
| B. obeum A2-162 | 2.94 | HFGFD-Tr | C-Tr |
| C. amycolatum SK46 | 2.71 | HFGFD-V | V-Tr |
| P. vulgatus str. 3975 RP4 | 2.70 | HFGFD-V | V-C |
| T. othiniensis | 2.63 | HFGFD-V | V-C |

C

**FIG 7**

FIG 8

A

B

C

**FIG 9**

FIG 10

FIG 11

FIG 12

**FIG 13**

**FIG 13 (continued)**

FIG 14

FIG 15

B

FIG 15 (continued)

FIG 16

FIG 17

FIG 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017134240 A
- US 2019117709 A1
- EP 3690026 A2

### Non-patent literature cited in the description

- **ZHENG et al.** *Int J Syst Evol Microbiol*, 2020, vol. 70 (4), 2782-2858
- **DUNCAN et al.** *Int J Syst Evol Microbiol.*, 2002, vol. 52, 2141-2146
- **EECKHAUT et al.** *Int J Syst Evol Microbiol*, 2008, vol. 58, 2799-2802
- **DUNCAN et al.** *Int J Syst Evol Microbiol.*, 2006, vol. 56, 2437-2441
- **DERRIEN et al.** *Int J Syst Evol Microbiol.*, 2004, vol. 54, 1469-1476
- **WALTER**. *Appl Environ Microbiol*, 2008, vol. 74, 4985-4996
- **SCHWIERTZ et al.** *Syst Appl Microbiol*, 2002, vol. 25, 46-51
- **VITAL et al.** *mBio*, 2014, vol. 5 (2), e00889-14
- **REICHARDT et al.** *The ISME Journal*, 2014, vol. 8, 1323-1335
- Remington: The Science and Practice of Pharmacy. Elsevier, 2020
- **ESLAM M et al.** A new definition for metabolic dysfunction-associated fatty liver disease: an international expert consensus statement. *J Hepatol.*, 2020, vol. 73, 202-209
- **LACKNER C.** *Nonalcoholic Fatty Liver Disease. Practical Hepatic Pathology: a Diagnostic Approach*, 2018, 167-187
- **KLEINEN et al.** Design and Validation of a Histological Scoring System for Nonalcoholic Fatty Liver Disease. *Hepatology*, 2005, vol. 41 (6), 1313-1321
- **XIA X.** Bioinformatics and the Cell: Modern Computational Approaches in Genomics, Proteomics and transcriptomics. Springer International Publishing, 2018
- **MOUNT D.** Bioinformatics: Sequence and Genome Analysis. Cold Spring Harbor Laboratory Press, 2004
- **HENIKOFF** ; **HENIKOFF**. *PNAS*, 1992, vol. 89, 915-919
- **ALTSCHUL et al.** *J. Mol. Biol.*, 1990, vol. 215, 403-10
- **ALTSCHUL et al.** *Nucleic Acids Res.*, 1997, vol. 25 (17), 3389-3402
- **GARCIA-LEZANA, T et al.** Restoration of a Healthy Intestinal Microbiota Normalizes Portal Hypertension in a Rat Model of Nonalcoholic Steatohepatitis. *HEPATOLOGY*, 2018, vol. 67 (4)
- **THOENDEL M et al.** *Journal of Clinical Microbiology*, 2020, vol. 58 (3), e00981-19
- **FRANZOSA EA et al.** Species-level functional profiling of metagenomes and metatranscriptomes. *Nature Methods*, 2018, vol. 15 (11), 962-8
- **MCMURDIE PJ** ; **HOLMES S**. *PLoS ONE*, 2013, vol. 8 (4), e61217
- **ROHART F.** *PLOS Computational Biology*, 2017, vol. 13 (11), e1005752
- **LIAO Y et al.** *Bioinformatics*, 2014, vol. 30 (7), 923-30
- **ANDERS S** ; **HUBER W.** *Genome Biology*, 2010, vol. 11 (10), R106
- **THÉVENOT EA**. *Journal of Proteome Research*, 2015, vol. 14 (8), 3322-35
- **WU T et al.** *The Innovation*, 2021, vol. 2 (3), 100141
- **MARTENS M et al.** *Nucleic Acids Research*, 2021, vol. 49 (D1), D613-21
- **AIBAR S et al.** SCENIC: Single-cell regulatory network inference and clustering. *Nature Methods*, 2017, vol. 14 (11), 1083-6
- **FUJII, M. et al.** *Med. Mol. Morphol.*, 2013, vol. 46, 141-152
- **YOUNOSSI ZM** ; **GOLABI P** ; **DE AVILA L** ; **PAIK JM** ; **SRISHORD M** ; **FUKUI N et al.** The global epidemiology of NAFLD and NASH in patients with type 2 diabetes: A systematic review and meta-analysis. *J. Hepatol.*, 2019, vol. 71, 793-801
- **QIAN Y** ; **FAN JG**. Obesity, fatty liver and liver cancer. Springer, 2018
- **LI J** ; **ZOU B** ; **YEO YH** ; **FENG Y** ; **XIE X** ; **LEE DH et al.** Prevalence, incidence, and outcome of non-alcoholic fatty liver disease in Asia, 1999-2019: a systematic review and meta-analysis. *Lancet Gastroenterol. Hepatol.*, 2019, vol. 4, 389-398

- **YOUNOSSI ZM** ; **TAMPI R** ; **PRIYADARSHINI M** ; **NADER F** ; **YOUNOSSI IM** ; **RACILA A**. Burden of Illness and Economic Model for Patients With Nonalcoholic Steatohepatitis in the United States. *Hepatology*, 2019, vol. 69, 564-572
- **CHALASANI N** ; **YOUNOSSI Z** ; **LAVINE JE** ; **CHARLTON M** ; **CUSI K** ; **RINELLA M et al.** The diagnosis and management of nonalcoholic fatty liver disease: Practice guidance from the American Association for the Study of Liver Diseases. *Hepatology*, 2018, vol. 67, 328-357
- **LAGO RM** ; **SINGH PP** ; **NESTO RW**. Congestive heart failure and cardiovascular death in patients with prediabetes and type 2 diabetes given thiazolidinediones: a meta-analysis of randomised clinical trials. *Lancet (London, England)*, 2007, vol. 370, 1129-1136
- **LINCOFF AM** ; **WOLSKI K** ; **NICHOLLS SJ** ; **NISSEN SE**. Pioglitazone and risk of cardiovascular events in patients with type 2 diabetes mellitus: a meta-analysis of randomized trials. *JAMA*, 2007, vol. 298, 1180-1188
- **NISSEN SE** ; **WOLSKI K**. Effect of rosiglitazone on the risk of myocardial infarction and death from cardiovascular causes. *N. Engl. J. Med.*, 2007, vol. 356, 2457-2471
- **GARCIA-LEZANA T** ; **RAURELL I** ; **BRAVO M** ; **TORRES-ARAUZ M** ; **SALCEDO MT** ; **SANTIAGO A et al.** Restoration of a healthy intestinal microbiota normalizes portal hypertension in a rat model of nonalcoholic steatohepatitis. *Hepatology*, 2018, vol. 67, 1485-1498